# EUROPEAN PATENT APPLICATION

(11) **EP 1 723 964 A1**
(43) Date of publication of application: **22.11.2006**
(21) Application number: 05291086.6
(22) Date of filing: 20.05.2005
(51) Int. Cl.: A61K 39/095, A61P 31/04, C07K 14/22, C07K 16/12, C12N 15/31, G01N 33/569, A61K 31/713

(54) **Use of penicillin-binding proteins or polynucleotides or antibodies thereof for preventing or treating bacterial infections**

(71) Applicant: INSTITUT PASTEUR, 75015 Paris (FR)
(72) Inventor: Zarantonelli, Maria Leticia, 75015 Paris (FR); Antignac, Aude, 35410 Chateaugiron (FR); Alonso, Jean-Michel, 78180 Montigny le Bretonneux (FR); Taha, Muhamed-Kheir, 94430 Chennevieres (FR)
(74) Representative: Warcoin, Jacques

(57) **Abstract**

The present invention is drawn to a penicillin-binding protein expressed by *Neisseria meningitidis,* or a fragment, or a variant, or a variant fragment thereof, or nucleic acids encoding them, or antibodies directed against them, as medicaments or in pharmaceutical compositions. The invention is also directed to the use of penicillin-binding proteins expressed by *Neisseria meningitidis,* or nucleic acids encoding them, or antibodies directed against them for the prophylactic and/or therapeutic immunotherapy of mammals, preferably human beings, against infection by *Neisseria meningitidis* and/or by a bacterial strain expressing a variant of a *Neisseria meningitidis* penicillin-binding protein.

SEQUENCE LISTING

<110> Institut Pasteur de Paris

<120> Use of penicillin-binding proteins or polynucleotides or
      antibodies thereof for preventing or treating bacterial
      infections

<130> D22995

<160> 13

<170> PatentIn version 3.1


<210> 1
<211> 798
<212> PRT
<213> Neisseiria meningitidis

<220>
<223> PBP1 protein of Neisseiria meningitidis (MC58)

<400> 1

Met Ile Lys Lys Ile Leu Thr Thr Cys Phe Gly Leu Val Phe Gly Phe
1               5                   10                  15

Cys Val Phe Gly Val Gly Leu Val Ala Ile Ala Ile Leu Val Thr Tyr
            20                  25                  30

Pro Lys Leu Pro Ser Leu Asp Ser Leu Gln His Tyr Gln Pro Lys Met
        35                  40                  45

Pro Leu Thr Ile Tyr Ser Ala Asp Gly Glu Val Ile Gly Met Tyr Gly
    50                  55                  60

Glu Gln Arg Arg Glu Phe Thr Lys Ile Gly Asp Phe Pro Glu Val Leu
65                  70                  75                  80

Arg Asn Ala Val Ile Ala Ala Glu Asp Lys Arg Phe Tyr Arg His Trp
                85                  90                  95

Gly Val Asp Val Trp Gly Val Ala Arg Ala Ala Val Gly Asn Val Val
            100                 105                 110

Ser Gly Ser Val Gln Ser Gly Ala Ser Thr Ile Thr Gln Gln Val Ala
        115                 120                 125

Lys Asn Phe Tyr Leu Ser Ser Glu Lys Thr Phe Thr Arg Lys Phe Asn
    130                 135                 140

Glu Val Leu Leu Ala Tyr Lys Ile Glu Gln Ser Leu Ser Lys Asp Lys
145                 150                 155                 160

Ile Leu Glu Leu Tyr Phe Asn Gln Ile Tyr Leu Gly Gln Arg Ala Tyr
                165                 170                 175

Gly Phe Ala Ser Ala Ala Gln Ile Tyr Phe Asn Lys Asn Val Arg Asp
            180                 185                 190

Leu Thr Leu Ala Glu Ala Ala Met Leu Ala Gly Leu Pro Lys Ala Pro
        195                 200                 205

Ser Ala Tyr Asn Pro Ile Val Asn Pro Glu Arg Ala Lys Leu Arg Gln
    210                 215                 220

Lys Tyr Ile Leu Asn Asn Met Leu Glu Glu Lys Met Ile Thr Val Gln
225                 230                 235                 240

Gln Arg Asp Gln Ala Leu Asn Glu Glu Leu His Tyr Glu Arg Phe Val
                245                 250                 255

Arg Lys Ile Asp Gln Ser Ala Leu Tyr Val Ala Glu Met Val Arg Gln
            260                 265                 270

Glu Leu Tyr Glu Lys Tyr Gly Glu Asp Ala Tyr Thr Gln Gly Phe Lys
        275                 280                 285

Val Tyr Thr Thr Val Arg Ala Asp His Gln Lys Val Ala Thr Glu Ala
    290                 295                 300

Leu Arg Lys Ala Leu Arg Asn Phe Asp Arg Gly Ser Ser Tyr Arg Gly
305                 310                 315                 320

Ala Glu Asn Tyr Ile Asp Leu Ser Lys Ser Glu Asp Val Glu Glu Thr
                325                 330                 335

Val Ser Gln Tyr Leu Ser Gly Leu Tyr Thr Val Asp Lys Met Val Pro
            340                 345                 350

Ala Val Val Leu Asp Val Thr Lys Lys Lys Asn Val Val Ile Gln Leu
        355                 360                 365

Pro Gly Gly Arg Arg Val Thr Leu Asp Arg Arg Ala Leu Gly Phe Ala
    370                 375                 380

Ala Arg Ala Val Asn Asn Glu Lys Met Gly Glu Asp Arg Ile Arg Arg
385                 390                 395                 400

Gly Ala Val Ile Arg Val Lys Asn Asn Gly Gly Arg Trp Ala Val Val
                405                 410                 415

Gln Glu Pro Leu Leu Gln Gly Ala Leu Val Ser Leu Asp Ala Lys Thr
            420                 425                 430

Gly Ala Val Arg Ala Leu Val Gly Gly Tyr Asp Phe His Ser Lys Thr
        435                 440                 445

Phe Asn Arg Ala Val Gln Ala Met Arg Gln Pro Gly Ser Thr Phe Lys
    450                 455                 460

Pro Phe Val Tyr Ser Ala Ala Leu Ser Lys Gly Met Thr Ala Ser Thr
465                 470                 475                 480

Val Val Asn Asp Ala Pro Ile Ser Leu Pro Gly Lys Gly Pro Asn Gly
                485                 490                 495

Ser Val Trp Thr Pro Lys Asn Ser Asp Gly Arg Tyr Ser Gly Tyr Ile
            500                 505                 510

Thr Leu Arg Gln Ala Leu Thr Ala Ser Lys Asn Met Val Ser Ile Arg

515                 520                 525

Ile Leu Met Ser Ile Gly Val Gly Tyr Ala Gln Gln Tyr Ile Arg Arg
    530                 535                 540

Phe Gly Phe Arg Ser Ser Glu Leu Pro Ala Ser Leu Ser Met Ala Leu
545                 550                 555                 560

Gly Thr Gly Glu Thr Thr Pro Leu Lys Val Ala Glu Ala Tyr Ser Val
                565                 570                 575

Phe Ala Asn Gly Gly Tyr Arg Val Ser Ser His Val Ile Asp Lys Ile
            580                 585                 590

Tyr Asp Arg Asp Gly Arg Leu Arg Ala Gln Met Gln Pro Leu Val Ala
        595                 600                 605

Gly Gln Asn Ala Pro Gln Ala Ile Asp Pro Arg Asn Ala Tyr Ile Met
    610                 615                 620

Tyr Lys Ile Met Gln Asp Val Val Arg Val Gly Thr Ala Arg Gly Ala
625                 630                 635                 640

Ala Ala Leu Gly Arg Thr Asp Ile Ala Gly Lys Thr Gly Thr Thr Asn
                645                 650                 655

Asp Asn Lys Asp Ala Trp Phe Val Gly Phe Asn Pro Asp Val Val Thr
            660                 665                 670

Ala Val Tyr Ile Gly Phe Asp Lys Pro Lys Ser Met Gly Arg Val Gly
        675                 680                 685

Tyr Gly Gly Thr Ile Ala Val Pro Val Trp Val Asp Tyr Met Arg Phe
    690                 695                 700

Ala Leu Lys Gly Lys Gln Gly Lys Gly Met Lys Met Pro Glu Gly Val
705                 710                 715                 720

Val Ser Ser Asn Gly Glu Tyr Tyr Met Lys Glu Arg Met Val Thr Asp
                725                 730                 735

Pro Gly Leu Thr Leu Asp Asn Ser Gly Ile Ala Pro Gln Pro Ser Arg
            740                 745                 750

Arg Ala Lys Glu Asp Asp Gly Gly Ala Ala Glu Gly Gly Arg Gln Ala
        755                 760                 765

Ala Asp Asp Glu Val Arg Gln Asp Met Gln Glu Thr Pro Val Leu Pro
    770                 775                 780

Ser Asn Thr Gly Ser Lys Gln Gln Gln Leu Asp Ser Leu Phe
785                 790                 795


<210> 2
<211> 798
<212> PRT
<213> Neisseiria meningitidis

<220>
<223> PBP1 protein of Neisseiria meningitidis (Z2491)

<400> 2

Met Ile Lys Lys Ile Leu Thr Thr Cys Phe Gly Leu Val Phe Gly Phe
1               5                   10                  15

Cys Val Phe Gly Val Gly Leu Val Ala Ile Ala Ile Leu Val Thr Tyr
            20                  25                  30

Pro Lys Leu Pro Ser Leu Asp Ser Leu Gln His Tyr Gln Pro Lys Met
        35                  40                  45

Pro Leu Thr Ile Tyr Ser Ala Asp Gly Glu Val Ile Gly Met Tyr Gly
    50                  55                  60

Glu Gln Arg Arg Glu Phe Thr Lys Ile Gly Asp Phe Pro Glu Val Leu
65                  70                  75                  80

Arg Asn Ala Val Ile Ala Ala Glu Asp Lys Arg Phe Tyr Arg His Trp
                85                  90                  95

Gly Val Asp Val Trp Gly Val Ala Arg Ala Ala Val Gly Asn Val Val
            100                 105                 110

Ser Gly Ser Val Gln Ser Gly Ala Ser Thr Ile Thr Gln Gln Val Ala
        115                 120                 125

Lys Asn Phe Tyr Leu Ser Ser Glu Lys Thr Phe Thr Arg Lys Phe Asn
    130                 135                 140

Glu Val Leu Leu Ala Tyr Lys Ile Glu Gln Ser Leu Ser Lys Asp Lys
145                 150                 155                 160

Ile Leu Glu Leu Tyr Phe Asn Gln Ile Tyr Leu Gly Gln Arg Ala Tyr
                165                 170                 175

Gly Phe Ala Ser Ala Ala Gln Ile Tyr Phe Asn Lys Asn Val Arg Asp
            180                 185                 190

Leu Thr Leu Ala Glu Ala Ala Met Leu Ala Gly Leu Pro Lys Ala Pro
        195                 200                 205

Ser Ala Tyr Asn Pro Ile Val Asn Pro Glu Arg Ala Lys Leu Arg Gln
    210                 215                 220

Lys Tyr Ile Leu Asn Asn Met Leu Glu Glu Lys Met Ile Thr Val Gln
225                 230                 235                 240

Gln Arg Asp Gln Ala Leu Asn Glu Glu Leu His Tyr Glu Arg Phe Val
                245                 250                 255

Arg Lys Ile Asp Gln Ser Ala Leu Tyr Val Ala Glu Met Val Arg Gln
            260                 265                 270

Glu Leu Tyr Glu Lys Tyr Gly Glu Asp Ala Tyr Thr Gln Gly Phe Lys
        275                 280                 285

Val Tyr Thr Thr Val Arg Ala Asp His Gln Lys Val Ala Thr Glu Ala
    290                 295                 300

Leu Arg Lys Ala Leu Arg Asn Phe Asp Arg Gly Ser Ser Tyr Arg Gly

305                 310                 315                 320

Ala Glu Asn Tyr Ile Asp Leu Ser Lys Ser Glu Asp Val Glu Glu Thr
                325                 330                 335

Val Ser Gln Tyr Leu Ser Gly Leu Tyr Thr Val Asp Lys Met Val Pro
            340                 345                 350

Ala Val Val Leu Asp Val Thr Lys Lys Lys Asn Val Val Ile Gln Leu
        355                 360                 365

Pro Gly Gly Arg Arg Val Thr Leu Asp Arg Arg Ala Leu Gly Phe Ala
    370                 375                 380

Ala Arg Ala Val Asn Asn Glu Lys Met Gly Glu Asp Arg Ile Arg Arg
385                 390                 395                 400

Gly Ala Val Ile Arg Val Lys Asn Asn Gly Gly Arg Trp Ala Val Val
                405                 410                 415

Gln Glu Pro Leu Leu Gln Gly Ala Leu Val Ser Leu Asp Ala Lys Thr
            420                 425                 430

Gly Ala Val Arg Ala Leu Val Gly Gly Tyr Asp Phe His Ser Lys Thr
        435                 440                 445

Phe Asn Arg Ala Val Gln Ala Met Arg Gln Pro Gly Ser Thr Phe Lys
    450                 455                 460

Pro Phe Val Tyr Ser Ala Ala Leu Ser Lys Gly Met Thr Ala Ser Thr
465                 470                 475                 480

Val Val Asn Asp Ala Pro Ile Ser Leu Pro Gly Lys Gly Pro Asn Gly
                485                 490                 495

Ser Val Trp Thr Pro Lys Asn Ser Asp Gly Arg Tyr Ser Gly Tyr Ile
            500                 505                 510

Thr Leu Arg Gln Ala Leu Thr Ala Ser Lys Asn Met Val Ser Ile Arg
        515                 520                 525

Ile Leu Met Ser Ile Gly Val Gly Tyr Ala Gln Gln Tyr Ile Arg Arg
    530                 535                 540

Phe Gly Phe Arg Ser Ser Glu Leu Pro Ala Ser Leu Ser Met Ala Leu
545                 550                 555                 560

Gly Thr Gly Glu Thr Thr Pro Leu Lys Val Ala Glu Ala Tyr Ser Val
                565                 570                 575

Phe Ala Asn Gly Gly Tyr Arg Val Ser Ser His Val Ile Asp Lys Ile
            580                 585                 590

Tyr Asp Arg Asp Gly Arg Leu Arg Ala Gln Met Gln Pro Leu Val Ala
        595                 600                 605

Gly Gln Asn Ala Pro Gln Ala Ile Asp Pro Arg Asn Ala Tyr Ile Met
    610                 615                 620

Tyr Lys Ile Met Gln Asp Val Val Arg Val Gly Thr Ala Arg Gly Ala
625                 630                 635                 640

Ala Ala Leu Gly Arg Thr Asp Ile Ala Gly Lys Thr Gly Thr Thr Asn
                645                 650                 655

Asp Asn Lys Asp Ala Trp Phe Val Gly Phe Asn Pro Asp Val Val Thr
            660                 665                 670

Ala Val Tyr Ile Gly Phe Asp Lys Pro Lys Ser Met Gly Arg Val Gly
        675                 680                 685

Tyr Gly Gly Thr Ile Ala Val Pro Val Trp Val Asp Tyr Met Arg Phe
    690                 695                 700

Ala Leu Lys Gly Lys Gln Gly Lys Gly Met Lys Met Pro Glu Gly Val
705                 710                 715                 720

Val Ser Ser Asn Gly Glu Tyr Tyr Met Lys Glu Arg Met Val Thr Asp
                725                 730                 735

Pro Gly Leu Thr Leu Asp Asn Ser Gly Ile Ala Pro Gln Pro Ser Arg
            740                 745                 750

Arg Ala Lys Glu Asp Asp Gly Gly Ala Ala Glu Gly Gly Arg Gln Ala
        755                 760                 765

Ala Asp Asp Glu Val Arg Gln Asp Met Gln Glu Thr Pro Val Leu Pro
    770                 775                 780

Ser Asn Thr Gly Ser Lys Gln Gln Gln Leu Asp Ser Leu Phe
785                 790                 795


<210> 3
<211> 581
<212> PRT
<213> Neisseiria meningitidis

<220>
<223> PBP2 protein of Neisseiria meningitidis (MC58)

<400> 3

Met Leu Ile Lys Ser Glu Tyr Lys Pro Arg Met Leu Pro Lys Glu Glu
1               5                   10                  15

Gln Val Lys Lys Pro Met Thr Ser Asn Gly Arg Ile Ser Phe Val Leu
            20                  25                  30

Met Ala Ile Ala Val Leu Phe Ala Gly Leu Ile Ala Arg Gly Leu Tyr
        35                  40                  45

Leu Gln Thr Val Thr Tyr Asn Phe Leu Lys Glu Gln Gly Asp Asn Arg
    50                  55                  60

Ile Val Arg Thr Gln Thr Leu Pro Ala Thr Arg Gly Thr Val Ser Asp
65                  70                  75                  80

Arg Asn Gly Ala Val Leu Ala Leu Ser Ala Pro Thr Glu Ser Leu Phe
                85                  90                  95

Ala Val Pro Lys Glu Met Lys Glu Met Pro Ser Ala Ala Gln Leu Glu

            100                 105                 110

Arg Leu Ser Glu Leu Val Asp Val Pro Val Asp Val Leu Arg Asn Lys
        115                 120                 125

Leu Glu Gln Lys Gly Lys Ser Phe Ile Trp Ile Lys Arg Gln Leu Asp
    130                 135                 140

Pro Lys Val Ala Glu Glu Val Lys Ala Leu Gly Leu Glu Asn Phe Val
145                 150                 155                 160

Phe Glu Lys Glu Leu Lys Arg His Tyr Pro Met Gly Asn Leu Phe Ala
                165                 170                 175

His Val Ile Gly Phe Thr Asp Ile Asp Gly Lys Gly Gln Glu Gly Leu
            180                 185                 190

Glu Leu Ser Leu Glu Asp Ser Leu His Gly Glu Asp Gly Ala Glu Val
        195                 200                 205

Val Leu Arg Asp Arg Gln Gly Asn Ile Val Asp Ser Leu Asp Ser Pro
    210                 215                 220

Arg Asn Lys Ala Pro Lys Asn Gly Lys Asp Ile Ile Leu Ser Leu Asp
225                 230                 235                 240

Gln Arg Ile Gln Thr Leu Ala Tyr Glu Glu Leu Asn Lys Ala Val Glu
                245                 250                 255

Tyr His Gln Ala Lys Ala Gly Thr Val Val Val Leu Asp Ala Arg Thr
            260                 265                 270

Gly Glu Ile Leu Ala Leu Ala Asn Thr Pro Ala Tyr Asp Pro Asn Arg
        275                 280                 285

Pro Gly Arg Ala Asp Ser Glu Gln Arg Arg Asn Arg Ala Val Thr Asp
    290                 295                 300

Met Ile Glu Pro Gly Ser Ala Ile Lys Pro Phe Val Ile Ala Lys Ala
305                 310                 315                 320

Leu Asp Ala Gly Lys Thr Asp Leu Asn Glu Arg Leu Asn Thr Gln Pro
                325                 330                 335

Tyr Lys Ile Gly Pro Ser Pro Val Arg Asp Thr His Val Tyr Pro Ser
            340                 345                 350

Leu Asp Val Arg Gly Ile Met Gln Lys Ser Ser Asn Val Gly Thr Ser
        355                 360                 365

Lys Leu Ser Ala Arg Phe Gly Ala Glu Glu Met Tyr Asp Phe Tyr His
    370                 375                 380

Glu Leu Gly Ile Gly Val Arg Met His Ser Gly Phe Pro Gly Glu Thr
385                 390                 395                 400

Ala Gly Leu Leu Arg Asn Trp Arg Arg Trp Arg Pro Ile Glu Gln Ala
                405                 410                 415

Thr Met Ser Phe Gly Tyr Gly Leu Gln Leu Ser Leu Leu Gln Leu Ala
            420                 425                 430

Arg Ala Tyr Thr Ala Leu Thr His Asp Gly Val Leu Leu Pro Val Ser
        435                 440                 445

Phe Glu Lys Gln Ala Val Ala Pro Gln Gly Lys Arg Ile Phe Lys Glu
    450                 455                 460

Ser Thr Ala Arg Glu Val Arg Asn Leu Met Val Ser Val Thr Glu Pro
465                 470                 475                 480

Gly Gly Thr Gly Thr Ala Gly Ala Val Asp Gly Phe Asp Val Gly Ala
                485                 490                 495

Lys Thr Gly Thr Ala Arg Lys Phe Val Asn Gly Arg Tyr Ala Asp Asn
            500                 505                 510

Lys His Ile Ala Thr Phe Ile Gly Phe Ala Pro Ala Lys Asn Pro Arg
        515                 520                 525

Val Ile Val Ala Val Thr Ile Asp Glu Pro Thr Ala His Gly Tyr Tyr
    530                 535                 540

Gly Gly Val Val Ala Gly Pro Pro Phe Lys Lys Ile Met Gly Gly Ser
545                 550                 555                 560

Leu Asn Ile Leu Gly Ile Ser Pro Thr Lys Pro Leu Thr Ala Ala Ala
                565                 570                 575

Val Lys Thr Pro Ser
            580


<210> 4
<211> 581
<212> PRT
<213> Neisseiria meningitidis

<220>
<223> PBP2 protein of Neisseiria meningitidis (Z2491)

<400> 4

Met Leu Ile Lys Ser Glu Tyr Lys Pro Arg Met Leu Pro Lys Glu Glu
1               5                   10                  15

Gln Val Lys Lys Pro Met Thr Ser Asn Gly Arg Ile Ser Phe Val Leu
            20                  25                  30

Met Ala Ile Ala Val Leu Phe Ala Gly Leu Ile Ala Arg Gly Leu Tyr
        35                  40                  45

Leu Gln Thr Val Thr Tyr Asn Phe Leu Lys Glu Gln Gly Asp Asn Arg
    50                  55                  60

Ile Val Arg Thr Gln Thr Leu Pro Ala Thr Arg Gly Thr Val Ser Asp
65                  70                  75                  80

Arg Asn Gly Ala Val Leu Ala Leu Ser Ala Pro Thr Glu Ser Leu Phe
                85                  90                  95

Ala Val Pro Lys Glu Met Lys Glu Met Pro Ser Ala Ala Gln Leu Glu

            100                 105                 110

Arg Leu Ser Glu Leu Val Asp Val Pro Val Asp Val Leu Arg Asn Lys
        115                 120                 125

Leu Glu Gln Lys Gly Lys Ser Phe Ile Trp Ile Lys Arg Gln Leu Asp
    130                 135                 140

Pro Lys Val Ala Glu Glu Val Lys Ala Leu Gly Leu Glu Asn Phe Val
145                 150                 155                 160

Phe Glu Lys Glu Leu Lys Arg His Tyr Pro Met Gly Asn Leu Phe Ala
                165                 170                 175

His Val Ile Gly Phe Thr Asp Ile Asp Gly Lys Gly Gln Glu Gly Leu
            180                 185                 190

Glu Leu Ser Leu Glu Asp Ser Leu His Gly Glu Asp Gly Ala Glu Val
        195                 200                 205

Val Leu Arg Asp Arg Gln Gly Asn Ile Val Asp Ser Leu Asp Ser Pro
    210                 215                 220

Arg Asn Lys Ala Pro Lys Asn Gly Lys Asp Ile Ile Leu Ser Leu Asp
225                 230                 235                 240

Gln Arg Ile Gln Thr Leu Ala Tyr Glu Glu Leu Asn Lys Ala Val Glu
                245                 250                 255

Tyr His Gln Ala Lys Ala Gly Thr Val Val Val Leu Asp Ala Arg Thr
            260                 265                 270

Gly Glu Ile Leu Ala Leu Ala Asn Thr Pro Ala Tyr Asp Pro Asn Arg
        275                 280                 285

Pro Gly Arg Ala Asp Ser Glu Gln Arg Arg Asn Arg Ala Val Thr Asp
    290                 295                 300

Met Ile Glu Pro Gly Ser Ala Ile Lys Pro Phe Val Ile Ala Lys Ala
305                 310                 315                 320

Leu Asp Ala Gly Lys Thr Asp Leu Asn Glu Arg Leu Asn Thr Gln Pro
                325                 330                 335

Tyr Lys Ile Gly Pro Ser Pro Val Arg Asp Thr His Val Tyr Pro Ser
            340                 345                 350

Leu Asp Val Arg Gly Ile Met Gln Lys Ser Ser Asn Val Gly Thr Ser
        355                 360                 365

Lys Leu Ser Ala Arg Phe Gly Ala Glu Glu Met Tyr Asp Phe Tyr His
    370                 375                 380

Glu Leu Gly Ile Gly Val Arg Met His Ser Gly Phe Pro Gly Glu Thr
385                 390                 395                 400

Ala Gly Leu Leu Arg Asn Trp Arg Arg Trp Arg Pro Ile Glu Gln Ala
                405                 410                 415

Thr Met Ser Phe Gly Tyr Gly Leu Gln Leu Ser Leu Leu Gln Leu Ala
            420                 425                 430

Arg Ala Tyr Thr Ala Leu Thr His Asp Gly Val Leu Leu Pro Val Ser
        435                 440                 445

Phe Glu Lys Gln Ala Val Ala Pro Gln Gly Lys Arg Ile Phe Lys Glu
    450                 455                 460

Ser Thr Ala Arg Glu Val Arg Asn Leu Met Val Ser Val Thr Glu Pro
465                 470                 475                 480

Gly Gly Thr Gly Thr Ala Gly Ala Val Asp Gly Phe Asp Val Gly Ala
                485                 490                 495

Lys Thr Gly Thr Ala Arg Lys Phe Val Asn Gly Arg Tyr Ala Asp Asn
            500                 505                 510

Lys His Ile Ala Thr Phe Ile Gly Phe Ala Pro Ala Lys Asn Pro Arg
        515                 520                 525

Val Ile Val Ala Val Thr Ile Asp Glu Pro Thr Ala His Gly Tyr Tyr
    530                 535                 540

Gly Gly Val Val Ala Gly Pro Pro Phe Lys Lys Ile Met Gly Gly Ser
545                 550                 555                 560

Leu Asn Ile Leu Gly Ile Ser Pro Thr Lys Pro Leu Thr Ala Ala Ala
                565                 570                 575

Val Lys Thr Pro Ser
            580

<210> 5
<211> 469
<212> PRT
<213> Neisseiria meningitidis

<220>
<223> PBP3 protein of Neisseiria meningitidis (MC58)

<400> 5

Met Asn Phe Pro Lys Thr Ala Ala Ser Leu Leu Leu Leu Leu Ala Ser
1               5                   10                  15

Leu Ala Ala His Ala Leu Asp Thr Gly Arg Ile Pro Gln Asn Glu Ile
            20                  25                  30

Ala Val Tyr Val Gln Glu Leu Asp Ser Gly Lys Val Ile Ile Asp His
        35                  40                  45

Arg Ser Asp Val Pro Val Asn Pro Ala Ser Thr Met Lys Leu Val Thr
    50                  55                  60

Ala Phe Ala Ala Phe Lys Thr Phe Gly Ser Asn Tyr Arg Trp Ala Thr
65                  70                  75                  80

Glu Phe Lys Ser Asn Gly Thr Val Asn Asp Gly Thr Leu Asp Gly Asn
                85                  90                  95

Leu Tyr Trp Ala Gly Ser Gly Asp Pro Val Phe Asn Gln Glu Asn Leu
            100                 105                 110

Leu Asp Ala Gln Lys Gln Leu Arg Glu Gln Gly Ile Leu Asn Ile Thr
        115                 120                 125

Gly His Leu Met Leu Asp His Ser Leu Trp Gly Glu Val Gly Ser Pro
    130                 135                 140

Asp Asp Phe Glu Ala Asp Ser Gly Ser Pro Phe Met Thr Pro Pro Asn
145                 150                 155                 160

Pro Thr Met Leu Ser Ala Gly Met Val Met Val Arg Ala Glu Arg Asn
                165                 170                 175

Ala Ala Gly Ser Thr Asp Ile Leu Thr Asp Pro Pro Leu Pro His Ile
            180                 185                 190

Phe Ala Gln Asn Asn Leu Lys Ile Thr Ala Ser Gln Ala Ala Cys Pro
        195                 200                 205

Ser Ile Lys Lys Leu Met Arg Ala Ser Phe Ser Asp Asn Thr Leu Lys
    210                 215                 220

Leu Arg Gly Asn Ile Pro Glu Ser Cys Leu Gly Lys Pro Val Gly Val
225                 230                 235                 240

Arg Met Phe Ala Leu Asp Glu Leu Ile Arg Gln Ser Phe Thr Asn His
                245                 250                 255

Trp Leu Leu Gly Gly Gly Arg Ile Ser Asp Gly Ile Gly Ile Ala Asp
            260                 265                 270

Thr Pro Glu Gly Ala Gln Thr Leu Ala Val Ala His Ala Lys Pro Met
        275                 280                 285

Lys Glu Ile Leu Thr Asp Met Asn Lys Arg Ser Asp Asn Leu Ile Ala
    290                 295                 300

Arg Ser Val Phe Leu Lys Leu Gly Gly Asp Gly Lys Leu Pro Ala Val
305                 310                 315                 320

Ser Glu Gln Ala Ala Ser Ala Val Arg Arg Glu Leu Ala Val Ser Gly
                325                 330                 335

Ile Asp Val Ala Asp Leu Val Leu Glu Asn Gly Ser Gly Leu Ser Arg
            340                 345                 350

Lys Glu Arg Val Thr Ala Arg Met Met Ala Gln Met Leu Glu Thr Ala
        355                 360                 365

Tyr Phe Ser Pro Phe Ala Gln Asp Phe Ile Asp Thr Leu Pro Ile Ala
    370                 375                 380

Gly Thr Asp Gly Thr Leu Arg Asn Arg Phe Lys Gln Ser Gly Gly Leu
385                 390                 395                 400

Leu Arg Leu Lys Thr Gly Thr Leu Asn Asn Val Arg Ala Leu Ala Gly
                405                 410                 415

Tyr Trp Leu Gly Asp Lys Pro Met Ala Val Val Val Ile Ile Asn Ser
            420                 425                 430

Gly Arg Ala Val Ser Leu Leu Pro Asp Leu Asp Asn Phe Val Ala Asn
        435                 440                 445

Asn Ile Ile Ser Gly Gly Asp Gly Trp Leu Asp Ala Lys Leu Met Cys
    450                 455                 460

Lys Glu Arg Arg Ala
465

<210> 6
<211> 312
<212> PRT
<213> Neisseiria meningitidis

<220>
<223> PBP4 prtein of Neisseiria meningitidis (MC58)

<400> 6

Met Ser Ile His Thr Leu Lys Arg Leu Pro Ser Ser Leu Leu Leu Gly
1               5                   10                  15

Leu Cys Leu Ser Leu Pro Ser Ala His Leu Phe Ala Asp Asn Asp Ile
            20                  25                  30

Leu Gly Gln Phe Leu Glu Gln Asn Met Leu Thr Ser Ser Asp Pro Ile
        35                  40                  45

Glu Ile Phe Ala Glu Ser Thr Ile His Pro Thr Asn Thr Gln Ala Ile
    50                  55                  60

Thr Gly Gly Leu Ile Leu Ser Ser Gln Ser Ala Leu Val Val Asn Asn
65                  70                  75                  80

Lys Thr Gly Gln Ile Leu Tyr Gln Lys Asn Ala Asp Arg Ile Met Pro
                85                  90                  95

Ile Ala Ser Ile Ser Lys Leu Met Ser Ala Met Val Val Leu Asp Ala
            100                 105                 110

Asn Leu Asp Met Asn Glu Thr Val Thr Ile Thr Pro Asp Glu Ile Asp
        115                 120                 125

Arg Ile Lys Gly Thr Gly Ser Arg Leu Ala Ile Gly Thr Ala Leu Thr
    130                 135                 140

Arg Lys Lys Leu Leu His Leu Ser Leu Met Ser Ser Glu Asn Arg Ala
145                 150                 155                 160

Thr His Ala Leu Gly Arg Thr Tyr Pro Gly Gly Met Gly Ala Phe Val
                165                 170                 175

Ala Ala Met Asn Arg Lys Ala Gln Ser Leu Gly Met Tyr Gly Ser Arg
            180                 185                 190

Phe Tyr Glu Pro Thr Gly Leu Asn Phe Gln Asn Val Ser Thr Ala Lys
        195                 200                 205

Asp Leu Ser Leu Met Val Asn Ala Ala Ala Gln Tyr Pro Gln Ile Arg
    210                 215                 220

Thr Asn Ser Thr Ser Asn Tyr Ala Ser Val Gln Thr Lys Asn Gly Gln
225                 230                 235                 240

Gln Asn Tyr Lys Asn Ser Asn Ala Leu Val Arg Glu Gly Met Trp Asn
                245                 250                 255

Ile Glu Leu Gln Lys Thr Gly Tyr Ile Arg Glu Ala Gly Arg Ser Met
            260                 265                 270

Val Val Lys Ala Asn Ile Gln Asn Gln Pro Val Thr Ile Val Leu Leu
        275                 280                 285

Asn Ser Pro Thr Ser Ala Thr Arg Val Asn Asp Ala Arg Lys Ile Glu
    290                 295                 300

Ser Trp Met Leu Gln Gln Arg Ser
305                 310

<210> 7
<211> 312
<212> PRT
<213> Neisseiria meningitidis

<220>
<223> PBP4 protein of Neisseiria meningitidis (Z2491)

<400> 7

Met Ser Ile His Thr Leu Lys Arg Leu Pro Ser Ser Leu Leu Leu Gly
1               5                   10                  15

Leu Cys Leu Ser Leu Pro Ser Ala His Leu Phe Ala Asp Asn Asp Ile
            20                  25                  30

Leu Gly Gln Phe Leu Glu Gln Asn Met Leu Thr Ser Ser Asp Pro Ile
        35                  40                  45

Glu Ile Phe Ala Glu Ser Thr Ile His Pro Thr Asn Thr Gln Ala Ile
    50                  55                  60

Thr Gly Gly Leu Ile Leu Ser Ser Gln Ser Ala Leu Val Val Asn Asn
65                  70                  75                  80

Lys Thr Gly Gln Ile Leu Tyr Gln Lys Asn Ala Asp Arg Ile Met Pro
                85                  90                  95

Ile Ala Ser Ile Ser Lys Leu Met Ser Ala Met Val Val Leu Asp Ala
            100                 105                 110

Asn Leu Asp Met Asn Glu Thr Val Thr Ile Thr Pro Asp Glu Ile Asp
        115                 120                 125

Arg Ile Lys Gly Thr Gly Ser Arg Leu Ala Ile Gly Thr Ala Leu Thr
    130                 135                 140

Arg Lys Glu Leu Leu His Leu Ser Leu Met Ser Ser Glu Asn Arg Ala
145                 150                 155                 160

Thr His Ala Leu Gly Arg Thr Tyr Pro Gly Gly Met Gly Ala Phe Val

165                 170                 175

Ala Ala Met Asn Arg Lys Ala Gln Ser Leu Gly Met Tyr Gly Ser Arg
            180                 185                 190

Phe Tyr Glu Pro Thr Gly Leu Asn Phe Gln Asn Val Ser Thr Ala Lys
        195                 200                 205

Asp Leu Ser Leu Met Val Asn Ala Ala Ala Gln Tyr Pro Gln Ile Arg
    210                 215                 220

Thr Asn Ser Thr Ser Asn Tyr Ala Ser Val Gln Thr Lys Asn Gly Gln
225                 230                 235                 240

Gln Asn Tyr Lys Asn Ser Asn Ala Leu Val Arg Glu Gly Met Trp Asn
                245                 250                 255

Ile Glu Leu Gln Lys Thr Gly Tyr Ile Arg Glu Ala Gly Arg Ser Met
            260                 265                 270

Val Val Lys Ala Asn Ile Gln Asn Gln Pro Val Thr Ile Val Leu Leu
        275                 280                 285

Asn Ser Pro Thr Ser Ala Thr Arg Val Asn Asp Ala Arg Lys Ile Glu
    290                 295                 300

Ser Trp Met Leu Gln Gln Arg Ser
305                 310

<210> 8
<211> 389
<212> PRT
<213> Neisseiria meningitidis

<220>
<223> PBP5 protein of Neisseiria meningitidis (MC58)

<400> 8

Met Thr Ala His Lys Ile Leu Pro Val Leu Leu Ser Ile Ile Leu Gly
1               5                   10                  15

Val Ser His Ala Thr Ala Ala Ser Pro Ala Pro Asn Arg Pro Thr Val
            20                  25                  30

His Ala Ala Pro Thr Phe Gln Thr Pro Glu Thr Leu Thr Ala Ala His
        35                  40                  45

Ile Val Ile Asp Leu Gln Ser Lys Gln Ile Leu Ser Ala Lys Asn Ile
    50                  55                  60

Asn Thr Pro Val Glu Pro Ala Ala Leu Thr Gln Leu Met Thr Ala Tyr
65                  70                  75                  80

Leu Val Phe Lys Asn Met Lys Ser Gly Asn Ile Gln Ser Glu Glu Asn
                85                  90                  95

Leu Lys Ile Pro Glu Ser Ala Trp Ala Ser Glu Gly Ser Arg Met Phe

            100                 105                 110

Val Arg Pro Gly Asp Thr Val Ser Thr Asp Lys Leu Leu Lys Gly Met
        115                 120                 125

Ile Ala Leu Ser Ala Asn Asp Ala Ala Leu Thr Leu Ala Gly Arg Leu
    130                 135                 140

Gly Asn Gly Ser Ile Glu Asn Phe Val Gln Gln Met Asn Lys Glu Ala
145                 150                 155                 160

Arg Arg Leu Gly Met Lys Asn Thr Val Phe Lys Asn Pro Thr Gly Leu
                165                 170                 175

Ser Arg Glu Gly Gln Val Ser Thr Ala Lys Asp Leu Ala Leu Leu Ser
            180                 185                 190

Glu Ala Leu Met Arg Asp Phe Pro Glu Tyr Tyr Pro Leu Phe Ser Ile
        195                 200                 205

Lys Ser Phe Lys Phe Lys Asn Ile Glu Gln Asn Asn Arg Asn Ile Leu
    210                 215                 220

Leu Tyr Arg Asp Asn Asn Val Asn Gly Leu Lys Ala Gly His Thr Glu
225                 230                 235                 240

Ser Gly Gly Tyr Asn Leu Ala Val Ser Tyr Ser Gly Asn Gly Arg His
                245                 250                 255

Ile Leu Val Ile Thr Leu Gly Ser Glu Ser Ala Glu Thr Arg Ala Ser
            260                 265                 270

Asp Asn Ser Lys Leu Leu Asn Trp Ala Leu Gln Ala Phe Asp Thr Pro
        275                 280                 285

Lys Ile Tyr Pro Lys Gly Lys Thr Val Ala Gln Ile Gln Ile Ser Gly
    290                 295                 300

Gly Ser Lys Lys Thr Val Arg Ala Gly Phe Leu Lys Glu Ala Tyr Ile
305                 310                 315                 320

Thr Leu Pro His Lys Glu Ala Lys Met Ala Glu Gln Ile Leu Glu Thr
                325                 330                 335

Ile Gln Pro Ile Pro Ala Pro Val Lys Lys Gly Gln Ile Leu Gly Lys
            340                 345                 350

Ile Lys Ile Arg Gln Asn Gly Tyr Thr Ile Ala Glu Lys Glu Ile Val
        355                 360                 365

Ala Leu Glu Asn Val Lys Lys Arg Ser Arg Trp Gln Arg Leu Trp Ala
    370                 375                 380

Cys Leu Thr Gly Gln
385

<210> 9
<211> 798
<212> PRT
<213> Neisseiria meningitidis

<220>
<223> PBP1 protein of Neisseiria gonorrhoeae (FA 1090)

<400> 9

Met Ile Lys Lys Ile Leu Thr Thr Cys Phe Gly Leu Phe Phe Gly Phe
1               5                   10                  15

Cys Val Phe Gly Val Gly Leu Val Ala Ile Ala Ile Leu Val Thr Tyr
            20                  25                  30

Pro Lys Leu Pro Ser Leu Asp Ser Leu Gln His Tyr Gln Pro Lys Met
        35                  40                  45

Pro Leu Thr Ile Tyr Ser Ala Asp Gly Glu Val Ile Gly Met Tyr Gly
    50                  55                  60

Glu Gln Arg Arg Glu Phe Thr Lys Ile Gly Asp Phe Pro Glu Val Leu
65                  70                  75                  80

Arg Asn Ala Val Ile Ala Ala Glu Asp Lys Arg Phe Tyr Arg His Trp
                85                  90                  95

Gly Val Asp Val Trp Gly Val Ala Arg Ala Ala Val Gly Asn Val Val
            100                 105                 110

Ser Gly Ser Val Gln Ser Gly Ala Ser Thr Ile Thr Gln Gln Val Ala
        115                 120                 125

Lys Asn Phe Tyr Leu Ser Ser Glu Lys Thr Phe Thr Arg Lys Phe Asn
    130                 135                 140

Glu Val Leu Leu Ala Tyr Lys Ile Glu Gln Ser Leu Ser Lys Asp Lys
145                 150                 155                 160

Ile Leu Glu Leu Tyr Phe Asn Gln Ile Tyr Leu Gly Gln Arg Ala Tyr
                165                 170                 175

Gly Phe Ala Ser Ala Ala Gln Ile Tyr Phe Asn Lys Asn Val Arg Asp
            180                 185                 190

Leu Thr Leu Ala Glu Ala Ala Met Leu Ala Gly Leu Pro Lys Ala Pro
        195                 200                 205

Ser Ala Tyr Asn Pro Ile Val Asn Pro Glu Arg Ala Lys Leu Arg Gln
    210                 215                 220

Lys Tyr Ile Leu Asn Asn Met Leu Glu Glu Lys Met Ile Thr Val Gln
225                 230                 235                 240

Gln Arg Asp Gln Ala Leu Asn Glu Glu Leu His Tyr Glu Arg Phe Val
                245                 250                 255

Arg Lys Ile Asp Gln Ser Ala Leu Tyr Val Ala Glu Met Val Arg Arg
            260                 265                 270

Glu Leu Tyr Glu Lys Tyr Gly Glu Asp Ala Tyr Thr Gln Gly Phe Lys
        275                 280                 285

Val Tyr Thr Thr Val Arg Thr Asp His Gln Lys Ala Ala Thr Glu Ala

290                 295                 300

Leu Arg Lys Ala Leu Arg Asn Phe Asp Arg Gly Ser Ser Tyr Arg Gly
305                 310                 315                 320

Ala Glu Asn Tyr Ile Asp Leu Ser Lys Ser Glu Asp Val Glu Glu Thr
                325                 330                 335

Val Ser Gln Tyr Leu Ser Gly Leu Tyr Thr Val Asp Lys Met Val Pro
            340                 345                 350

Ala Val Val Leu Asp Val Thr Lys Lys Lys Asn Val Val Ile Gln Leu
        355                 360                 365

Pro Gly Gly Arg Arg Val Ala Leu Asp Arg Arg Ala Leu Gly Phe Ala
    370                 375                 380

Ala Arg Ala Val Asp Asn Glu Lys Met Gly Glu Asp Arg Ile Arg Arg
385                 390                 395                 400

Gly Ala Val Ile Arg Val Lys Asn Asn Gly Gly Arg Trp Ala Val Val
                405                 410                 415

Gln Glu Pro Leu Leu Gln Gly Ala Leu Val Ser Leu Asp Ala Lys Thr
            420                 425                 430

Gly Ala Val Arg Ala Leu Val Gly Gly Tyr Asp Phe His Ser Lys Thr
        435                 440                 445

Phe Asn Arg Ala Val Gln Ala Met Arg Gln Pro Gly Ser Thr Phe Lys
    450                 455                 460

Pro Phe Val Tyr Ser Ala Ala Leu Ser Lys Gly Met Thr Ala Ser Thr
465                 470                 475                 480

Val Val Asn Asp Ala Pro Ile Ser Leu Pro Gly Lys Gly Pro Asn Gly
                485                 490                 495

Ser Val Trp Thr Pro Lys Asn Ser Asp Gly Arg Tyr Ser Gly Tyr Ile
            500                 505                 510

Thr Leu Arg Gln Ala Leu Thr Ala Ser Lys Asn Met Val Ser Ile Arg
        515                 520                 525

Ile Leu Met Ser Ile Gly Val Gly Tyr Ala Gln Gln Tyr Ile Arg Arg
    530                 535                 540

Phe Gly Phe Arg Pro Ser Glu Leu Pro Ala Ser Leu Ser Met Ala Leu
545                 550                 555                 560

Gly Thr Gly Glu Thr Thr Pro Leu Lys Val Ala Glu Ala Tyr Ser Val
                565                 570                 575

Phe Ala Asn Gly Gly Tyr Arg Val Ser Ser His Val Ile Asp Lys Ile
            580                 585                 590

Tyr Asp Arg Asp Gly Arg Leu Arg Ala Gln Met Gln Pro Leu Val Ala
        595                 600                 605

Gly Gln Asn Ala Pro Gln Ala Ile Asp Pro Arg Asn Ala Tyr Ile Met
    610                 615                 620

Tyr Lys Ile Met Gln Asp Val Val Arg Val Gly Thr Ala Arg Gly Ala
625                 630                 635                 640

Ala Ala Leu Gly Arg Thr Asp Ile Ala Gly Lys Thr Gly Thr Thr Asn
                645                 650                 655

Asp Asn Lys Asp Ala Trp Phe Val Gly Phe Asn Pro Asp Val Val Thr
            660                 665                 670

Ala Val Tyr Ile Gly Phe Asp Lys Pro Lys Ser Met Gly Arg Ala Gly
        675                 680                 685

Tyr Gly Gly Thr Ile Ala Val Pro Val Trp Val Asp Tyr Met Arg Phe
    690                 695                 700

Ala Leu Lys Gly Lys Gln Gly Lys Gly Met Lys Met Pro Glu Gly Val
705                 710                 715                 720

Val Ser Ser Asn Gly Glu Tyr Tyr Met Lys Glu Arg Met Val Thr Asp
                725                 730                 735

Pro Gly Leu Met Leu Asp Asn Ser Gly Ile Ala Pro Gln Pro Ser Arg
            740                 745                 750

Arg Ala Lys Glu Asp Asp Glu Ala Ala Val Glu Asn Glu Gln Gln Gly
        755                 760                 765

Arg Ser Asp Glu Thr Arg Gln Asp Val Gln Glu Thr Pro Val Leu Pro
    770                 775                 780

Ser Asn Thr Asp Ser Lys Gln Gln Gln Leu Asp Ser Leu Phe
785                 790                 795


<210> 10
<211> 821
<212> PRT
<213> Neisseiria meningitidis

<220>
<223> PBP1b protein of Streptococcus pneumoniae (R6)

<400> 10

Met Gln Asn Gln Leu Asn Glu Leu Lys Arg Lys Met Leu Glu Phe Phe
1               5                   10                  15

Gln Gln Lys Gln Lys Asn Lys Lys Ser Ala Arg Pro Gly Lys Lys Gly
            20                  25                  30

Ser Ser Thr Lys Lys Ser Lys Thr Leu Asp Lys Ser Val Ile Phe Pro
        35                  40                  45

Ala Ile Leu Leu Ser Ile Lys Ala Leu Phe Asn Leu Leu Phe Val Leu
    50                  55                  60

Gly Phe Leu Gly Gly Met Leu Gly Ala Gly Ile Ala Leu Gly Tyr Gly
65                  70                  75                  80

Val Ala Leu Phe Asp Lys Val Arg Val Pro Gln Thr Glu Glu Leu Val

                85                  90                  95

Asn Gln Val Lys Asp Ile Ser Ser Ile Ser Glu Ile Thr Tyr Ser Asp
            100                 105                 110

Gly Thr Val Ile Ala Ser Ile Glu Ser Asp Leu Leu Arg Thr Ser Ile
        115                 120                 125

Ser Ser Glu Gln Ile Ser Glu Asn Leu Lys Lys Ala Ile Ile Ala Thr
    130                 135                 140

Glu Asp Glu His Phe Lys Glu His Lys Gly Val Val Pro Lys Ala Val
145                 150                 155                 160

Ile Arg Ala Thr Leu Gly Lys Phe Val Gly Leu Gly Ser Ser Ser Gly
                165                 170                 175

Gly Ser Thr Leu Thr Gln Gln Leu Ile Lys Gln Gln Val Val Gly Asp
            180                 185                 190

Ala Pro Thr Leu Ala Arg Lys Ala Ala Glu Ile Val Asp Ala Leu Ala
        195                 200                 205

Leu Glu Arg Ala Met Asn Lys Asp Glu Ile Leu Thr Thr Tyr Leu Asn
    210                 215                 220

Val Ala Pro Phe Gly Arg Asn Asn Lys Gly Gln Asn Ile Ala Gly Ala
225                 230                 235                 240

Arg Gln Ala Ala Glu Gly Ile Phe Gly Val Asp Ala Ser Gln Leu Thr
                245                 250                 255

Val Pro Gln Ala Ala Phe Leu Ala Gly Leu Pro Gln Ser Pro Ile Thr
            260                 265                 270

Tyr Ser Pro Tyr Glu Asn Thr Gly Glu Leu Lys Ser Asp Glu Asp Leu
        275                 280                 285

Glu Ile Gly Leu Arg Arg Ala Lys Ala Val Leu Tyr Ser Met Tyr Arg
    290                 295                 300

Thr Gly Ala Leu Ser Lys Asp Glu Tyr Ser Gln Tyr Lys Asp Tyr Asp
305                 310                 315                 320

Leu Lys Gln Asp Phe Leu Pro Ser Gly Thr Val Thr Gly Ile Ser Arg
                325                 330                 335

Asp Tyr Leu Tyr Phe Thr Thr Leu Ala Glu Ala Gln Glu Arg Met Tyr
            340                 345                 350

Asp Tyr Leu Ala Gln Arg Asp Asn Val Ser Ala Lys Glu Leu Lys Asn
        355                 360                 365

Glu Ala Thr Gln Lys Phe Tyr Arg Asp Leu Ala Ala Lys Glu Ile Glu
    370                 375                 380

Asn Gly Gly Tyr Lys Ile Thr Thr Thr Ile Asp Gln Lys Ile His Ser
385                 390                 395                 400

Ala Met Gln Ser Ala Val Ala Asp Tyr Gly Tyr Leu Leu Asp Asp Gly
                405                 410                 415

Thr Gly Arg Val Glu Val Gly Asn Val Leu Met Asp Asn Gln Thr Gly
            420                 425                 430

Ala Ile Leu Gly Phe Val Gly Gly Arg Asn Tyr Gln Glu Asn Gln Asn
        435                 440                 445

Asn His Ala Phe Asp Thr Lys Arg Ser Pro Ala Ser Thr Thr Lys Pro
    450                 455                 460

Leu Leu Ala Tyr Gly Ile Ala Ile Asp Gln Gly Leu Met Gly Ser Glu
465                 470                 475                 480

Thr Ile Leu Ser Asn Tyr Pro Thr Asn Phe Ala Asn Gly Asn Pro Ile
                485                 490                 495

Met Tyr Ala Asn Ser Lys Gly Thr Gly Met Met Thr Leu Gly Glu Ala
            500                 505                 510

Leu Asn Tyr Ser Trp Asn Ile Pro Ala Tyr Trp Thr Tyr Arg Met Leu
        515                 520                 525

Arg Glu Lys Gly Val Asp Val Lys Gly Tyr Met Glu Lys Met Gly Tyr
    530                 535                 540

Glu Ile Pro Glu Tyr Gly Ile Glu Ser Leu Pro Met Gly Gly Gly Ile
545                 550                 555                 560

Glu Val Thr Val Ala Gln His Thr Asn Gly Tyr Gln Thr Leu Ala Asn
                565                 570                 575

Asn Gly Val Tyr His Gln Lys His Val Ile Ser Lys Ile Glu Ala Ala
            580                 585                 590

Asp Gly Arg Val Val Tyr Glu Tyr Gln Asp Lys Pro Val Gln Val Tyr
        595                 600                 605

Ser Lys Ala Thr Ala Thr Ile Met Gln Gly Leu Leu Arg Glu Val Leu
    610                 615                 620

Ser Ser Arg Val Thr Thr Thr Phe Lys Ser Asn Leu Thr Ser Leu Asn
625                 630                 635                 640

Pro Thr Leu Ala Asn Ala Asp Trp Ile Gly Lys Thr Gly Thr Thr Asn
                645                 650                 655

Gln Asp Glu Asn Met Trp Leu Met Leu Ser Thr Pro Arg Leu Thr Leu
            660                 665                 670

Gly Gly Trp Ile Gly His Asp Asp Asn His Ser Leu Ser Arg Arg Ala
        675                 680                 685

Gly Tyr Ser Asn Asn Ser Asn Tyr Met Ala His Leu Val Asn Ala Ile
    690                 695                 700

Gln Gln Ala Ser Pro Ser Ile Trp Gly Asn Glu Arg Phe Ala Leu Asp
705                 710                 715                 720

Pro Ser Val Val Lys Ser Glu Val Leu Lys Ser Thr Gly Gln Lys Pro
                725                 730                 735

Glu Lys Val Ser Val Glu Gly Lys Glu Val Glu Val Thr Gly Ser Thr
            740                 745                 750

Val Thr Ser Tyr Trp Ala Asn Lys Ser Gly Ala Pro Ala Thr Ser Tyr
        755                 760                 765

Arg Phe Ala Ile Gly Gly Ser Asp Ala Asp Tyr Gln Asn Ala Trp Ser
    770                 775                 780

Ser Ile Val Gly Ser Leu Pro Thr Pro Ser Ser Ser Ser Ser Ser Ser
785                 790                 795                 800

Ser Ser Ser Ser Asp Ser Ser Asn Ser Ser Thr Thr Arg Pro Ser Ser
                805                 810                 815

Ser Arg Ala Arg Arg
            820


<210> 11
<211> 731

<212> PRT
<213> Neisseiria meningitidis

<220>
<223> PBP2a protein of Streptococcus pneumoniae (R6)

<400> 11

Met Lys Leu Asp Lys Leu Phe Glu Lys Phe Leu Ser Leu Phe Lys Lys
1               5                   10                  15

Glu Thr Ser Glu Leu Glu Asp Ser Asp Ser Thr Ile Leu Arg Arg Ser
            20                  25                  30

Arg Ser Asp Arg Lys Lys Leu Ala Gln Val Gly Pro Ile Arg Lys Phe
        35                  40                  45

Trp Arg Arg Tyr His Leu Thr Lys Ile Ile Leu Ile Leu Gly Leu Ser
    50                  55                  60

Ala Gly Leu Leu Val Gly Ile Tyr Leu Phe Ala Val Ala Lys Ser Thr
65                  70                  75                  80

Asn Val Asn Asp Leu Gln Asn Ala Leu Lys Thr Arg Thr Leu Ile Phe
                85                  90                  95

Asp Arg Glu Glu Lys Glu Ala Gly Ala Leu Ser Gly Gln Lys Gly Thr
            100                 105                 110

Tyr Val Glu Leu Thr Asp Ile Ser Lys Asn Leu Gln Asn Ala Val Ile
        115                 120                 125

Ala Thr Glu Asp Arg Ser Phe Tyr Lys Asn Asp Gly Ile Asn Tyr Gly
    130                 135                 140

Arg Phe Phe Leu Ala Ile Val Thr Ala Gly Arg Ser Gly Gly Gly Ser
145                 150                 155                 160

Thr Ile Thr Gln Gln Leu Ala Lys Asn Ala Tyr Leu Ser Gln Asp Gln

                165                 170                 175

Thr Val Glu Arg Lys Ala Lys Glu Phe Phe Leu Ala Leu Glu Leu Ser
            180                 185                 190

Lys Lys Tyr Ser Lys Glu Gln Ile Leu Thr Met Tyr Leu Asn Asn Ala
        195                 200                 205

Tyr Phe Gly Asn Gly Val Trp Gly Val Glu Asp Ala Ser Lys Lys Tyr
    210                 215                 220

Phe Gly Val Ser Ala Ser Glu Val Ser Leu Asp Gln Ala Ala Thr Leu
225                 230                 235                 240

Ala Gly Met Leu Lys Gly Pro Glu Leu Tyr Asn Pro Leu Asn Ser Val
                245                 250                 255

Glu Asp Ser Thr Asn Arg Arg Asp Thr Val Leu Gln Asn Met Val Ala
            260                 265                 270

Ala Gly Tyr Ile Asp Lys Asn Gln Glu Thr Lys Ala Ala Glu Val Asp
        275                 280                 285

Met Thr Ser Gln Leu His Asp Lys Tyr Glu Gly Lys Ile Ser Asp Tyr
    290                 295                 300

Arg Tyr Pro Ser Tyr Phe Asp Ala Val Val Asn Glu Ala Val Ser Lys
305                 310                 315                 320

Tyr Asn Leu Thr Glu Glu Glu Ile Val Asn Asn Gly Tyr Arg Ile Tyr
                325                 330                 335

Thr Glu Leu Asp Gln Asn Tyr Gln Ala Asn Met Gln Ile Val Tyr Glu
            340                 345                 350

Asn Thr Ser Leu Phe Pro Arg Ala Glu Asp Gly Thr Phe Ala Gln Ser
        355                 360                 365

Gly Ser Val Ala Leu Glu Pro Lys Thr Gly Gly Val Arg Gly Val Val
    370                 375                 380

Gly Gln Val Ala Asp Asn Asp Lys Thr Gly Phe Arg Asn Phe Asn Tyr
385                 390                 395                 400

Ala Thr Gln Ser Lys Arg Ser Pro Gly Ser Thr Ile Lys Pro Leu Val
                405                 410                 415

Val Tyr Thr Pro Ala Val Glu Ala Ser Trp Ala Leu Asn Lys Gln Leu
            420                 425                 430

Asp Asn His Thr Met Gln Tyr Asp Ser Tyr Lys Val Asp Asn Tyr Ala
        435                 440                 445

Gly Ile Lys Thr Ser Arg Glu Val Pro Met Tyr Gln Ala Leu Ala Glu
    450                 455                 460

Ser Leu Asn Leu Pro Ala Val Ala Thr Val Asn Asp Leu Gly Val Asp
465                 470                 475                 480

Lys Ala Phe Glu Ala Gly Glu Lys Phe Gly Leu Asn Met Glu Lys Val
                485                 490                 495

Asp Arg Val Leu Gly Val Ala Leu Gly Ser Gly Val Glu Thr Asn Pro
            500                 505                 510

Leu Gln Met Ala Gln Ala Tyr Ala Ala Phe Ala Asn Glu Gly Leu Met
        515                 520                 525

Pro Glu Ala His Phe Ile Ser Arg Ile Glu Asn Ala Ser Gly Gln Val
    530                 535                 540

Ile Ala Ser His Lys Asn Ser Gln Lys Arg Val Ile Asp Lys Ser Val
545                 550                 555                 560

Ala Asp Lys Met Thr Ser Met Met Leu Gly Thr Phe Thr Asn Gly Thr
                565                 570                 575

Gly Ile Ser Ser Ser Pro Ala Asp Tyr Val Met Ala Gly Lys Thr Gly
            580                 585                 590

Thr Thr Glu Ala Val Phe Asn Pro Glu Tyr Thr Ser Asp Gln Trp Val
        595                 600                 605

Ile Gly Tyr Thr Pro Asp Val Val Ile Ser His Trp Leu Gly Phe Pro
    610                 615                 620

Thr Thr Asp Glu Asn His Tyr Leu Ala Gly Ser Thr Ser Asn Gly Ala
625                 630                 635                 640

Ala His Val Phe Arg Asn Ile Ala Asn Thr Ile Leu Pro Tyr Thr Pro
                645                 650                 655

Gly Ser Thr Phe Thr Val Glu Asn Ala Tyr Lys Gln Asn Gly Ile Ala
            660                 665                 670

Pro Ala Asn Thr Lys Arg Gln Val Gln Thr Asn Asp Asn Ser Gln Thr
        675                 680                 685

Asp Asp Asn Leu Ser Asp Ile Arg Gly Arg Ala Gln Ser Leu Val Asp
    690                 695                 700

Glu Ala Ser Arg Ala Ile Ser Asp Ala Lys Ile Lys Glu Lys Ala Gln
705                 710                 715                 720

Thr Ile Trp Asp Ser Ile Val Asn Leu Phe Arg
                725                 730

<210> 12
<211> 582
<212> PRT
<213> Neisseiria meningitidis

<220>
<223> PBP2 protein of Neisseiria gonorrhoeae (FA 1090)

<400> 12

Met Leu Ile Lys Ser Glu Tyr Lys Pro Arg Met Leu Pro Lys Glu Glu
1               5                   10                  15

Gln Val Lys Lys Pro Met Thr Ser Asn Gly Arg Ile Ser Phe Val Leu
            20                  25                  30

Met Ala Met Ala Val Leu Phe Ala Cys Leu Ile Ala Arg Gly Leu Tyr
        35                  40                  45

Leu Gln Thr Val Thr Tyr Asn Phe Leu Lys Glu Gln Gly Asp Asn Arg
    50                  55                  60

Ile Val Arg Thr Gln Ala Leu Pro Ala Thr Arg Gly Thr Val Ser Asp
65                  70                  75                  80

Arg Asn Gly Ala Val Leu Ala Leu Ser Ala Pro Thr Glu Ser Leu Phe
                85                  90                  95

Ala Val Pro Lys Asp Met Lys Glu Met Pro Ser Ala Ala Gln Leu Glu
            100                 105                 110

Arg Leu Ser Glu Leu Val Asp Val Pro Val Asp Val Leu Arg Asn Lys
        115                 120                 125

Leu Glu Gln Lys Gly Lys Ser Phe Ile Trp Ile Lys Arg Gln Leu Asp
    130                 135                 140

Pro Lys Val Ala Glu Glu Val Lys Ala Leu Gly Leu Glu Asn Phe Val
145                 150                 155                 160

Phe Glu Lys Glu Leu Lys Arg His Tyr Pro Met Gly Asn Leu Phe Ala
                165                 170                 175

His Val Ile Gly Phe Thr Asp Ile Asp Gly Lys Gly Gln Glu Gly Leu
            180                 185                 190

Glu Leu Ser Leu Glu Asp Ser Leu Tyr Gly Glu Asp Gly Ala Glu Val
        195                 200                 205

Val Leu Arg Asp Arg Gln Gly Asn Ile Val Asp Ser Leu Asp Ser Pro
    210                 215                 220

Arg Asn Lys Ala Pro Gln Asn Gly Lys Asp Ile Ile Leu Ser Leu Asp
225                 230                 235                 240

Gln Arg Ile Gln Thr Leu Ala Tyr Glu Glu Leu Asn Lys Ala Val Glu
                245                 250                 255

Tyr His Gln Ala Lys Ala Gly Thr Val Val Val Leu Asp Ala Arg Thr
            260                 265                 270

Gly Glu Ile Leu Ala Leu Ala Asn Thr Pro Ala Tyr Asp Pro Asn Arg
        275                 280                 285

Pro Gly Arg Ala Asp Ser Glu Gln Arg Arg Asn Arg Ala Val Thr Asp
    290                 295                 300

Met Ile Glu Pro Gly Ser Ala Ile Lys Pro Phe Val Ile Ala Lys Ala
305                 310                 315                 320

Leu Asp Ala Gly Lys Thr Asp Leu Asn Glu Arg Leu Asn Thr Gln Pro
                325                 330                 335

Tyr Lys Ile Gly Pro Ser Pro Val Arg Asp Asp Thr His Val Tyr Pro

340                 345                 350

Ser Leu Asp Val Arg Gly Ile Met Gln Lys Ser Ser Asn Val Gly Thr
        355                 360                 365

Ser Lys Leu Ser Ala Arg Phe Gly Ala Glu Glu Met Tyr Asp Phe Tyr
    370                 375                 380

His Glu Leu Gly Ile Gly Val Arg Met His Ser Gly Phe Pro Gly Glu
385                 390                 395                 400

Thr Ala Gly Leu Leu Arg Asn Trp Arg Arg Trp Arg Pro Ile Glu Gln
                405                 410                 415

Ala Thr Met Ser Phe Gly Tyr Gly Leu Gln Leu Ser Leu Leu Gln Leu
            420                 425                 430

Ala Arg Ala Tyr Thr Ala Leu Thr His Asp Gly Val Leu Leu Pro Leu
        435                 440                 445

Ser Phe Glu Lys Gln Ala Val Ala Pro Gln Gly Lys Arg Ile Phe Lys
    450                 455                 460

Glu Ser Thr Ala Arg Glu Val Arg Asn Leu Met Val Ser Val Thr Glu
465                 470                 475                 480

Pro Gly Gly Thr Gly Thr Ala Gly Ala Val Asp Gly Phe Asp Val Gly
                485                 490                 495

Ala Lys Thr Gly Thr Ala Arg Lys Phe Val Asn Gly Arg Tyr Ala Asp
            500                 505                 510

Asn Lys His Val Ala Thr Phe Ile Gly Phe Ala Pro Ala Lys Asn Pro
        515                 520                 525

Arg Val Ile Val Ala Val Thr Ile Asp Glu Pro Thr Ala His Gly Tyr
    530                 535                 540

Tyr Gly Gly Val Val Ala Gly Pro Pro Phe Lys Lys Ile Met Gly Gly
545                 550                 555                 560

Ser Leu Asn Ile Leu Gly Ile Ser Pro Thr Lys Pro Leu Thr Ala Ala
                565                 570                 575

Ala Val Lys Thr Pro Ser
            580

<210> 13
<211> 750
<212> PRT
<213> Neisseiria meningitidis

<220>
<223> PBP2X protein of Streptococcus pneumoniae (R6)

<400> 13

Met Lys Trp Thr Lys Arg Val Ile Arg Tyr Ala Thr Lys Asn Arg Lys
1               5                   10                  15

Ser Pro Ala Glu Asn Arg Arg Arg Val Gly Lys Ser Leu Ser Leu Leu
            20                  25                  30

Ser Val Phe Val Phe Ala Ile Phe Leu Val Asn Phe Ala Val Ile Ile
        35                  40                  45

Gly Thr Gly Thr Arg Phe Gly Thr Asp Leu Ala Lys Glu Ala Lys Lys
    50                  55                  60

Val His Gln Thr Thr Arg Thr Val Pro Ala Lys Arg Gly Thr Ile Tyr
65                  70                  75                  80

Asp Arg Asn Gly Val Pro Ile Ala Glu Asp Ala Thr Ser Tyr Asn Val
                85                  90                  95

Tyr Ala Val Ile Asp Glu Asn Tyr Lys Ser Ala Thr Gly Lys Ile Leu
            100                 105                 110

Tyr Val Glu Lys Thr Gln Phe Asn Lys Val Ala Glu Val Phe His Lys
        115                 120                 125

Tyr Leu Asp Met Glu Glu Ser Tyr Val Arg Glu Gln Leu Ser Gln Pro
    130                 135                 140

Asn Leu Lys Gln Val Ser Phe Gly Ala Lys Gly Asn Gly Ile Thr Tyr
145                 150                 155                 160

Ala Asn Met Met Ser Ile Lys Lys Glu Leu Glu Ala Ala Glu Val Lys
                165                 170                 175

Gly Ile Asp Phe Thr Thr Ser Pro Asn Arg Ser Tyr Pro Asn Gly Gln
            180                 185                 190

Phe Ala Ser Ser Phe Ile Gly Leu Ala Gln Leu His Glu Asn Glu Asp
        195                 200                 205

Gly Ser Lys Ser Leu Leu Gly Thr Ser Gly Met Glu Ser Ser Leu Asn
    210                 215                 220

Ser Ile Leu Ala Gly Thr Asp Gly Ile Ile Thr Tyr Glu Lys Asp Arg
225                 230                 235                 240

Leu Gly Asn Ile Val Pro Gly Thr Glu Gln Val Ser Gln Arg Thr Met
                245                 250                 255

Asp Gly Lys Asp Val Tyr Thr Thr Ile Ser Ser Pro Leu Gln Ser Phe
            260                 265                 270

Met Glu Thr Gln Met Asp Ala Phe Gln Glu Lys Val Lys Gly Lys Tyr
        275                 280                 285

Met Thr Ala Thr Leu Val Ser Ala Lys Thr Gly Glu Ile Leu Ala Thr
    290                 295                 300

Thr Gln Arg Pro Thr Phe Asp Ala Asp Thr Lys Glu Gly Ile Thr Glu
305                 310                 315                 320

Asp Phe Val Trp Arg Asp Ile Leu Tyr Gln Ser Asn Tyr Glu Pro Gly
                325                 330                 335

Ser Thr Met Lys Val Met Met Leu Ala Ala Ala Ile Asp Asn Asn Thr
            340                 345                 350

Phe Pro Gly Gly Glu Val Phe Asn Ser Ser Glu Leu Lys Ile Ala Asp
        355                 360                 365

Ala Thr Ile Arg Asp Trp Asp Val Asn Glu Gly Leu Thr Gly Gly Arg
    370                 375                 380

Met Met Thr Phe Ser Gln Gly Phe Ala His Ser Ser Asn Val Gly Met
385                 390                 395                 400

Thr Leu Leu Glu Gln Lys Met Gly Asp Ala Thr Trp Leu Asp Tyr Leu
                405                 410                 415

Asn Arg Phe Lys Phe Gly Val Pro Thr Arg Phe Gly Leu Thr Asp Glu
            420                 425                 430

Tyr Ala Gly Gln Leu Pro Ala Asp Asn Ile Val Asn Ile Ala Gln Ser
        435                 440                 445

Ser Phe Gly Gln Gly Ile Ser Val Thr Gln Thr Gln Met Ile Arg Ala
    450                 455                 460

Phe Thr Ala Ile Ala Asn Asp Gly Val Met Leu Glu Pro Lys Phe Ile
465                 470                 475                 480

Ser Ala Ile Tyr Asp Pro Asn Asp Gln Thr Ala Arg Lys Ser Gln Lys
                485                 490                 495

Glu Ile Val Gly Asn Pro Val Ser Lys Asp Ala Ala Ser Leu Thr Arg
            500                 505                 510

Thr Asn Met Val Leu Val Gly Thr Asp Pro Val Tyr Gly Thr Met Tyr
        515                 520                 525

Asn His Ser Thr Gly Lys Pro Thr Val Thr Val Pro Gly Gln Asn Val
    530                 535                 540

Ala Leu Lys Ser Gly Thr Ala Gln Ile Ala Asp Glu Lys Asn Gly Gly
545                 550                 555                 560

Tyr Leu Val Gly Leu Thr Asp Tyr Ile Phe Ser Ala Val Ser Met Ser
                565                 570                 575

Pro Ala Glu Asn Pro Asp Phe Ile Leu Tyr Val Thr Val Gln Gln Pro
            580                 585                 590

Glu His Tyr Ser Gly Ile Gln Leu Gly Glu Phe Ala Asn Pro Ile Leu
        595                 600                 605

Glu Arg Ala Ser Ala Met Lys Asp Ser Leu Asn Leu Gln Thr Thr Ala
    610                 615                 620

Lys Ala Leu Glu Gln Val Ser Gln Gln Ser Pro Tyr Pro Met Pro Ser
625                 630                 635                 640

Val Lys Asp Ile Ser Pro Gly Asp Leu Ala Glu Glu Leu Arg Arg Asn
                645                 650                 655

Leu Val Gln Pro Ile Val Val Gly Thr Gly Thr Lys Ile Lys Asn Ser
            660                 665                 670

Ser Ala Glu Glu Gly Lys Asn Leu Ala Pro Asn Gln Gln Val Leu Ile
        675                 680                 685

Leu Ser Asp Lys Ala Glu Glu Val Pro Asp Met Tyr Gly Trp Thr Lys
    690                 695                 700

Glu Thr Ala Glu Thr Leu Ala Lys Trp Leu Asn Ile Glu Leu Glu Phe
705                 710                 715                 720

Gln Gly Ser Gly Ser Thr Val Gln Lys Gln Asp Val Arg Ala Asn Thr
                725                 730                 735

Ala Ile Lys Asp Ile Lys Lys Ile Thr Leu Thr Leu Gly Asp
            740                 745                 750

## Description

The present invention is drawn to a penicillin-binding protein expressed by *Neisseria meningitidis,* or a fragment, or a variant, or a variant fragment thereof, or nucleic acids encoding them, or antibodies or antibody fragments directed against them, as medicaments or in pharmaceutical compositions. The invention is also directed to the use of penicillin-binding proteins expressed by *Neisseria meningitidis,* or nucleic acids encoding them, or antibodies directed against them for the prophylactic and/or therapeutic immunotherapy of mammals, preferably human beings, against infection by *Neisseria meningitidis* and/or by a bacterial strain expressing a variant of a *Neisseria meningitidis* penicillin-binding protein.

Two complementary therapeutic strategies have greatly improved the capacity to resist bacterial infections: the use of antibiotics and the prophylactic or therapeutic vaccination of exposed individuals. However, although the combination of these two strategies allowed to almost eradicate some bacterial diseases, some difficulties aroused concerning other infections. On the one hand, an increasing number of bacterial strains developed resistance to the most common antibiotics, creating an endless competition between the conception of new antibiotics and the adaptation of bacteria to these new antibiotics. On the other hand, the elaboration of vaccines against some bacteria came up against the difficulty to find an efficient vaccine target and the genetic variability of potential protein targets.

In particular, the management of meningococcal infections requires immediate antibiotic therapy for the case and also chemoprophylaxis and vaccination (when available) of household contacts to prevent secondary cases and epidemics. Penicillin G is the antibiotic of choice against meningococcal disease (Quagliarello, V. J., and W. M. Scheld. 1997. N Engl J Med 336:708-16) but meningococcal strains with diminished susceptibility are increasingly recorded (Antignac, A., et al. 2003. Clin Infect Dis 37:912-20; Saez Nieto, et al. 1990. Lancet 336:54).

Current vaccines against *Neisseria meningitidis* are based on capsular polysaccharide and are available only against strains belonging to serogroups A, C, Y and W135 but not against strains of serogroup B. Protein candidates have been explored to develop vaccines against strains of serogroup B such as the major outer membrane porin (PorA) and the transferrin binding protein B (TbpB) (Claassen, I., et al. 1996. Vaccine 14:1001-8; Rokbi, B., et al. 1997. Infect Immun 65:55-63). Outer membrane vesicles-based vaccines were also developed (Fredriksen, J. H. et al. 1991. NIPH Ann 14:67-80; Holst, J., et al. 2003. Vaccine 21:734-7; Sierra, G. V., et al. 1991. NIPH Ann 14:195-210). However, this strategy is hindered by the high degree of variability of *Neisseiria meningitidis* due to horizontal DNA exchanges between strains, resulting in modification of structures of genetic loci (Maiden, M. C. 1993. FEMS Microbiol Lett 112:243-50).

There is thus a need for new vaccine candidates against bacterial infections, in particular *Neisseria meningitidis* infection. An ideal vaccine candidate molecule should be present in all strains of a particular bacterial species and have conserved regions that can be targeted by protective antibodies.

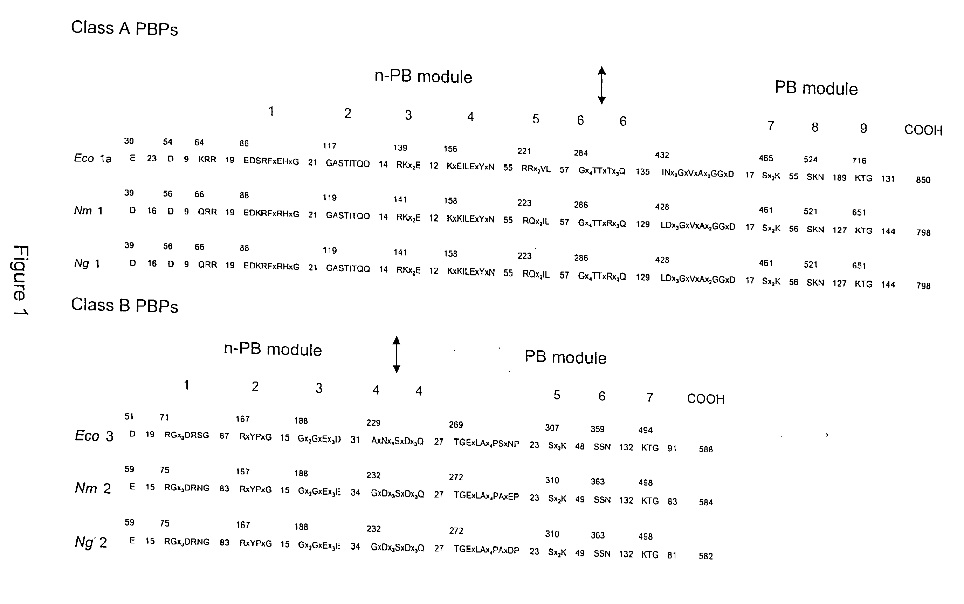

Penicillin-binding proteins (PBPs) are conserved proteins that are involved in the assembly and metabolism of the bacterial cell wall peptidoglycan (Goffin, C., and J. M. Ghuysen. 1998. Microbiol Mol Biol Rev. 62(4):1079-93). PBPS are constituted of two functional modules: an acyl serine transferase penicillin-binding (PB) module, which is linked to the carboxy end of a non-penicillin-binding (n-PB) module through a conserved fusion site. The two modules form a single polypeptide chain which folds on the exterior of the plasma membrane and is anchored by a transmembrane spanner. PBPs cluster into two classes, A and B. In each class, several conserved amino acid patterns are present both in the PB and n-PB modules (Figure 1). Outside these conserved amino acid patterns, a great variability can be observed among strains of several species such as *Neisseria meningitidis* and *Streptococcus pneumoniae.*

PBPs are also implicated in the mechanism of penicillin anti-bacterial action thanks to their ability to catalyze the rupture of the β-lactam amide bond of penicillin and the formation of a serine ester-linked penicilloyl enzyme, which is almost completely inert, thus unable to get involved in the biosynthesis of peptidoglycans. However, the use of β-lactam antibiotics resulted in the emergence and the spread of PBPs with a low or decreased affinity for the drug among important bacterial pathogens. This decreased affinity for penicillin is due to genetic alterations in the PB module (Goffin, C., and J. M. Ghuysen. Microbiol Mol Biol Rev. 62(4):1079-93).

The implication of PBPs in the resistance to β-lactam antibiotics has been thoroughly investigated, but their possible role as vaccine candidates has not been systematically analyzed at the present time, in particular in *Neisseiria meningitidis* for which it has never been even suggested. Three PBPs (PBP1, PBP2 and PBP3) have been reported in *Neisseiria meningitidis* (Barbour, A. G. 1981. Antimicrob Agents Chemother 19:316-22) but others were identified based on the genome sequence (Parkhill, J., et al. 2000. Nature 404:502-6; Tettelin, H., et al. 2000. Science 287:1809-15). PBP2, encoded by *penA* gene, is a 60 kDa protein, which is homologous to high-molecular weight class B PBPs and most likely catalyzes a transpeptidation reaction that is necessary for the cross-linking of peptidoglycan in the meningococcal cell wall. Highly related *penA*^{*S*} and different *penA*^{*I*} alleles were described in susceptible strains (Pen^{S}) and strains with reduced susceptibility to penicillin G (Pen^{I}), respectively (Antignac, A., et al. 2001. J Antimicrob Chemother 47:285-96). While the 5' half of *penA* is highly conserved, alterations in the 3' half of the gene are responsible for reduced susceptibility to penicillin G and occur through DNA transformation (Spratt, B. G. 1994. Science 264:388-93). The active serine residue Sx₂K, the SxN and the KxG (usually KTG) motifs, which are all located in the 3' transpeptidase-encoding region are usually conserved, but a high degree of polymorphism is observed in the surrounding sequences (Antignac, A., et al. 2003. J Biol Chem 278:31529-35; Antignac, A., et al. 2001. J Antimicrob Chemother 47:285-96).

Despite the high degree of polymorphism observed in the sequences surrounding the conserved active serine residue Sx₂K, the SxN and the KxG (usually KTG) motifs of *Neisseria meningitidis* PBP2, the inventors surprisingly found that this protein is immunogenic during *Neisseria meningitidis* human infection, and that immunization of mice with a PBP2 fragment induces anti-PBP2 antibodies. In addition, antibodies directed against a fragment of the PBP2 protein of a particular *Neisseria meningitidis* strain are able to bind PBP2 proteins of other *Neisseria meningitidis* strains, including strains of distinct serogroups and/or with different levels of susceptibility to antibiotics. Finally, the inventors also demonstrated that immunization of mice with a PBP2 fragment induces protective immunity in vaccinated mice, and that antibodies directed against a fragment of the PBP2 protein of a particular *Neisseria meningitidis* strain are able to protect mice from infection by the same *Neisseria meningitidis* strain or a mutant thereof expressing an altered PBP2 protein.

The present invention thus concerns a penicillin-binding protein expressed by *Neisseria meningitidis,* or a fragment, or a variant, or a variant fragment thereof, as a medicament.

By a "penicillin-binding protein (PBP)", it is intended to designate a protein of the penicilloyl serine transferase family, comprising an acyl serine transferase penicillin-binding (PB) module linked to the carboxy end of a non-penicillin-binding (n-PB) module through a conserved fusion site, and a transmembrane spanner permitting the anchorage in the membrane. Three PBP proteins (PBP1, PBP2 and PBP3) have been reported in *Neisseria meningitidis* (Barbour, A. G. 1981. Antimicrob Agents Chemother 19:316-22). The analysis of the genomic sequence of two *Neisseria meningitidis* strains further suggests the existence of two additional PBP proteins in *Neisseria meningitidis* (Parkhill J. et al. 2000. Nature 404(6777):502-6; Tettelin H. et al. 2000. Science 287(5459):1809-15; which are herein incorporated by reference). All these PBP proteins are included in the scope of the present invention. References of amino acid sequences of *Neisseria meningitidis* PBP proteins expressed by particular *Neisseria meningitidis* strains are displayed in following Table 1. These sequences are particularly included in the scope of the present invention, but should not be considered as limiting the invention, since *Neisseria meningitidis* PBP proteins expressed by any other mutant *Neisseria meningitidis* strain are also included in the scope of the present invention.

**Table 1. Particular amino acid sequences of Neisseria meningitidis PBP proteins**

| Name | Accession number (N.m. strain) | Sequence | PB module position (aa) | PB module conserved motifs |
|---|---|---|---|---|
| PBP1 | AAF42144 (MC58), CAB83943 (Z2491) | SEQ ID NO: 1, SEQ ID NO: 2 | 294-798 | STFK, SKN, KTG |
| PBP2 | AAF40852 (MC58), CAB85289 (Z2491) | SEQ ID NO: 3, SEQ ID NO: 4 | 240-584 | SAIK, SSN, KTG |
| PBP3 | AAF42135 (MC58) | SEQ ID NO:5 | 114-469 | SIKK, SDN, KTG |
| PBP4 putative | AAF41162 (MC58), CAB84232 (Z2491) | SEQ ID NO: 6, SEQ ID NO: 7 | 31-312 | SISK, SEN, KTG |
| Putative additional PBP | AAF41288 (MC58) | SEQ ID NO: 8 | Not determined | Not determined |

A "fragment" of a penicillin-binding protein of *Neisseria meningitidis* means a partial penicillin-binding protein, which has the capacity, upon immunization with said fragment, to generate protective immunity, preferably protective antibodies, against *Neisseria meningitidis* infection, or against infections by bacteria expressing a PBP protein comprising said fragment or a variant thereof. A PBP fragment according to the invention thus comprises at least a T cell or B cell epitope, preferably a B cell epitope, and is thus preferably at least 6, at least 8, at least 10, more preferably at least 12, at least 15, at least 20, at least 25, at least 30, at least 40, at least 50, at least 75, at least 100 amino acids long. In certain embodiments, said fragment comprises at least one of the 3 conserved amino acid motifs of the PB module (Sx₂K, SxN, and KxG) of said PBP protein, preferably 2 or 3 of the conserved amino acid motifs of the PB module. Said fragment may comprise the complete PB module of said penicillin-binding protein. In particular, the fragment may be a partial *Neisseria meningitidis* penicillin-binding protein lacking the N-terminal transmembrane domain, in particular a partial *Neisseria meningitidis* PBP2 protein lacking the N-terminal transmembrane domain.

The term "variant" of a penicillin-binding protein expressed by *Neisseria meningitidis* refers to a penicillin-binding protein, which may be modified in one or more amino acid positions, and which has the following fearures:
- (i): It has at least 25%, at least 30%, at least 40%, at least 50%, more preferably at least 60%, at least 70%, at least 80%, most preferably at least 90%, at least 95%, at least 97%, at least 98%, at least 99%, at least 99.5%, at least 99.9% identity with the PB module of the specifically described *Neisseria meningitidis* protein, and
- (ii): Its conserved (Sx₂K, SxN, KxG) motifs have at least 70%, at least 80%, at least 90%, or 100% of identity with conserved amino acid motifs (Sx₂K, SxN, and KxG) of the specifically described *Neisseria meningitidis* protein.

According to the invention, a variant of a *Neisseria meningitidis* PBP protein has the capacity, upon immunization with said variant, to generate protective immunity, preferably protective antibodies, against *Neisseria meningitidis* infection, or against infection by a bacterial strain expressing said variant.

Conserved motifs of most bacterial penicillin-binding proteins are well determined (see Goffin, C., and J. M. Ghuysen. Microbiol Mol Biol Rev. 62(4):1079-93 for amino acid positions and accession numbers). In case of a putative penicillin-binding protein, conserved motifs are preferably determined so that the Sx2K and SxN motifs are separated by at least 45 amino acids and the SxN and KxG (usually KTG) motifs are separated by at least 100 amino acids.

Preferably, a variant, as defined above, which has an identity with the PB module of the specifically described *Neisseria meningitidis* protein inferior to 50%, additionnally has a spatial three-dimensionnal (3D) structure that is similar to that of the specifically described *Neisseria meningitidis* protein.

A "percentage of sequence identity" is determined by comparing two optimally aligned sequences over a comparison window, where the portion of the peptide or polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity.

The definition of sequence identity given above is the definition that would use one of skill in the art. The definition by itself does not need the help of any algorithm, said algorithms being helpful only to achieve the optimal alignments of sequences, rather than the calculation of sequence identity. From the definition given above, it follows that there is only one well defined value for the sequence identity between two compared sequences, which value corresponds to the value obtained for the best or optimal alignment.

The percentage of sequence identity between 2 amino acid sequences may be determined by using with default parameters the Blastp 2.0 program provided by the National Center for Biotechnology Information (http://www.ncbi.nlm.nih.gov/blast/; Tatusova et al., "Blast 2 sequences - a new tool for comparing protein and nucleotide sequences", FEMS Microbiol. Lett. 174 : 247-250), which is habitually used by the inventors and in general by the skilled man for comparing and determining the identity between two sequences.

The "three-dimensionnal (3D) structure similarity" between a *Neisseria meningitidis* PBP protein and a putative variant can be evaluated according to the goodness of fit between the two polypeptides. Goodness of fit can be represented by a variety of parameters known in the art including, for example, the root mean square deviation (RMSD). A lower RMSD between structures correlates with a better fit compared to a higher RMSD between structures (see for example, Doucet and Weber, Computer-Aided Molecular Design : Theory and Applications, Academic Press, San Diego, CA (1996)). Polypeptides with a similar three-dimensionnal (3D) structure can be identified by comparing mean RMSD values for the backbones of the polypeptides. Preferably, a *Neisseria meningitidis* PBP protein and a variant thereof can have a mean backbone RMSD compared to each other that is less than about 5A or less than about 3A.

Particular examples of variants of *Neisseria meningitidis* PBP proteins that are particularly included in the scope of the invention are displayed in following Table 2, as well as references of amino acid sequences of these variants in particular bacterial strains. Once again, these sequences are particularly included in the scope of the present invention, but should not be considered as limiting the invention, since any other variant, as defined above, of a *Neisseria meningitidis* PBP protein is also included in the scope of the present invention.

**Table 2. Particular amino acid sequences of several Neisseria meningitidis PBP proteins variants**

| Name | Species | Accession number (strain) | Sequence | PB module position (aa) | PB module conserved motifs | N.m. closer PBP | % identity with PB module | % identity with (Sx₂K, SxN, and KTG) motifs |
|---|---|---|---|---|---|---|---|---|
| Ngo-PBP1 | *Neisseria gonorrhoeae* | AAW88860 (FA 1090) | SEQ ID NO:9 | 294-798 | STFK, SKN, KTG | PBP1 | 93 | 100 |
| Spn PBP1b | *Streptococcus pneumoniae* | CAA05302 (R6) | SEQ ID NO:10 | 395-821 | STTK, SWN, KTG | PBP1 | 30 | 80 |
| Spn-PBP2a | *Streptococcus pneumoniae* | CAA05303 (R6) | SEQ ID NO:11 | 340-731 | STIK, SLN, KTG | PBP1 | 30 | 80 |
| Ngo-PBP2 | *Neisseria gonorrhoeae* | AAW90178 (FA 1090) | SEQ ID NO:12 | 240-582 | SAIK, SSN, KTG | PBP2 | 99 | 100 |
| Spn-PBP2X | *Streptococcus pneumoniae* | AAK99108 (R6) | SEQ ID NO:13 | 266-750 | STMK, SSN, KSG | PBP2 | 27 | 70 |

According to the invention, a "variant fragment" of a *Neisseria meningitidis* PBP protein is a fragment, as defined above, of a variant, as defined above, which further has at least 25%, at least 30%, at least 40%, at least 50%, more preferably at least 60%, at least 70%, at least 80%, most preferably at least 90%, at least 95%, at least 97%, at least 98%, at least 99%, at least 99.5%, at least 99.9% identity with the corresponding *Neisseria meningitidis* PBP protein fragment.

According to the invention, such a penicillin-binding protein expressed by *Neisseria meningitidis,* or variant thereof, may or may not be implicated in β-lactam antibiotics resistance. Examples of β-lactam antibiotics to which bacterial strains may become resistant due to the expression of new or mutated penicillin-binding proteins include penicillin G, amoxicillin, ampicillin, and cephalosporins.

According to the invention, a PBP protein expressed by *Neisseria meningitidis,* or fragment, or variant or variant fragment thereof may be naturally occurring or man-made by peptide synthesis or conventional recombinant technologies. In particular, it may or may not display any of the posttranslational modifications displayed by a naturally occurring *Neisseria meningitidis* PBP protein, or fragment, or variant, or variant fragment thereof, such as glycosylation patterns. According to the invention, a *Neisseria meningitidis* PBP protein, or fragment, or variant or variant fragment thus include any polypeptide with the amino acid sequence of a *Neisseria meningitidis* PBP protein, or fragment, or variant, or variant fragment thereof.

It may be further modified to facilitate its production, purification, or to improve its stability, biodisponibility or efficiency as a medicament. For example, it may be coupled to a carbohydrate, a lipid moiety or modified in other ways, e.g. phosphorylated or hydroxylated. In particular, it may be glycosylated, the coupled carbohydrate moiety having molecular weights of 20-30 kD in the natural molecule. Coupling of said *Neisseria meningitidis* PBP, or fragment, or variant, or variant fragment to one or more moieties may for instance be due to a posttranslational modification of the polypeptide performed by an organism producing the polypeptide or a modification resulting from chemical synthesis.

Said PBP expressed by *Neisseria meningitidis,* or fragment, or variant, or variant fragment may also be fused to another polypeptide sequence, which may be one that results in an increased expression of the protein when expressed in an organism, or facilitates or improves the purification and recovery of the fusion protein from said organism in terms of a more easy and economical recovery, or improves the immunogenicity or pharmacokinetics of said *Neisseria meningitidis* PBP, or fragment, or variant, or variant fragment thereof.

In some cases, it may be advantageous to cleave the fusion protein so as to obtain a polypeptide which substantially solely comprises said *Neisseria meningitidis* PBP, or fragment, or variant, or variant fragment. In these cases, said *Neisseria meningitidis* PBP, or fragment, or variant, or variant fragment thereof is preferably fused to a polypeptide sequence which may be specifically recognized by a cleaving agent, e.g. a chemical such as cyanogen bromide, hydroxylamine and 2-nitro-5-thiocyanobenzoate, or an enzyme, e.g. a peptidase, proteinase or protease, e.g. trypsin, chlostripain, and staphylococcal protease or factor Xa

The present invention also concerns a nucleic acid encoding at least one penicillin-binding protein expressed by *Neisseria meningitidis,* or a fragment, or a variant, or a variant fragment thereof, as a medicament.

According to the invention, a nucleic acid encoding at least one penicillin-binding protein expressed by *Neisseria meningitidis,* or fragment, or variant, or variant fragment thereof, may be any kind of nucleic acid, including single-stranded DNA, double-stranded DNA, single-stranded RNA, double-stranded RNA, or a RNA/DNA hybrib. In a preferred embodiment, the nucleic acid is either double-stranded DNA or single-stranded RNA. In another embodiment, the nucleic acid is an antisens RNA.

Such a nucleic acid may encode either a single PBP protein expressed by *Neisseria meningitidis* or a fragment, or a variant, or a variant fragment thereof, or multiple distinct PBP proteins expressed by *Neisseria meningitidis* or fragments, or variant, or variants fragments thereof. It may further encode other polypeptides. In the nucleic acid, at least one encoded *Neisseria meningitidis* PBP or variant thereof may be fused to any other polypeptide.

In the case of DNA, said nucleic acid may be provided with operably linked start and stop codons, promoter and terminator regions, and optionally other regulating sequences, such as an enhancer. The promoter, and optionally the enhancer allow for the expression of the PBP, or fragment or variant or variant fragment thereof coding sequence in a host target cell, which may be a prokaryotic or eukaryotic host cell. In one preferred embodiment, the DNA nucleic acid includes a cell-specific promoter that permits substantial transcription of the DNA only in predetermined cells. The DNA nucleic acid may be engineered so as not to integrate into the host cell genome.

Such a DNA nucleic acid may be inserted into a plasmid of partly bacterial origin, and notably carrying a bacterial replication origin and a gene allowing its selection, such as a gene for resistance to an antibiotic. This plasmid may also be provided with a replication origin allowing it to replicate in the target cells of its host, for instance, in the case of muscle cells, the replication origin of the bovine papilloma virus. Many such plasmids are known from one skilled in the art and a great number are commercially available.

A nucleic acid according to the invention may be naked or inserted into any delivery system know by one skilled in the art. For instance, delivery systems include viral vectors, bacterial vectors, or chemical vectors. Examples of suitable viral vectors include herpes simplex viral vectors, vaccina or alpha-virus vectors and retroviruses, including lentiviruses, adenoviruses and adeno-associated viruses. Preferably, these vectors are replication defective virus vectors.

Examples of chemical vectors are liposomes, or oil-in-water emulsions. Alternatively, a nucleic acid according to the invention may be attached to at least one carrier molecule. Such a carrier molecule may be a molecule that facilitates the targeting of the nucleic acid to a particular cell type, in particular a ligand capable of binding to the surface of a target cell, such as a ligand of a membrane receptor or an antibody directed against a membrane receptor.

Alternatively, a carrier molecule according to the invention may be a nucleic binding molecule preferably capable to protect the nucleic acid against nuclease enzymes. For instance, a nucleic binding molecule may be a polycationic polymer, such as poly-L-lysine, poly-D-lysine, polyethyleneimine, polyamidoamine, or polyamine; or a nucleic binding protein, such as a histone protein, a protamine, ornithine, putrescine, spermidine, spermine, a transcription factor or a homeobox protein.

Still another type of carrier molecule may be a polypeptide that is able to direct the intracellular localisation of the nucleic acid in a host cell. For instance, it may be a signal polypeptide that directs the nucleic acid to the nucleus in an eukaryote host cell.

The present invention also concerns an antibody or an antibody fragment directed against a penicillin-binding protein expressed by *Neisseria meningitidis* or a fragment, or a variant, or a variant fragment thereof, as a medicament.

Said antibody or antibody fragment medicament may be used alone or in combination with other drugs. For instance, it may be used in combination with an antibiotic, said antibiotic being preferably chosen in the group of β-lactam antibiotics, in particular penicillin G, amoxicillin, ampicillin, and cephalosporins.

The term "antibody" includes a polypeptide encoded by an immunoglobulin gene or immunoglobulin genes, capable of specifically binding to an antigen or epitope, and expressed by a B lymphocyte or a B lymphocyte derived clone. The term "antibody" is used in the broadest sense. Therefore, an "antibody" can be naturally occurring or man-made by peptide synthesis or by the production of monoclonal antibodies by conventional hybridoma technology.

An "antibody fragment" is defined as at least a portion of the variable region of the immunoglobulin molecule that binds to its target,i.e., the antigen-binding region. Well-known "antibody fragments" include:
- (i): a Fab fragment: a monovalent fragment consisting of the VL, VH, CL and CHI domains;
- (ii): a F(ab')2 fragment: a bivalent fragment comprising two Fab fragments linked by a disulfide bridge at the hinge region;
- (iii): a Fd fragment consisting of the VH and CHI domains;
- (iv): a Fv fragment consisting of the VL and VH domains of a single arm of an antibody;
- (v): a dAb fragment (Ward et al., (1989) Nature 341:544-546), which consists of a VH domain; and
- (vi): an isolated complementary determining region (CDR).

Single chain antibodies are also included by reference in the term "antibody fragment".

Methods of immunization, producing and isolating antibodies (polyclonal and monoclonal) are known to those skilled in the art and described in the scientific and patent literature. Antibodies also can be generated in vitro, e.g., using recombinant antibody binding site expressing phage display libraries, in addition to the traditional in vivo methods using animals.

Said antibodies or antibody fragments may belong to any class of antibody, such as IgM, IgD, IgG, IgE or IgA antibodies, as well as all subclasses such as IgG1, IgG2, IgG3, IgG4.

Said antibody or antibody fragment may be polyclonal or monoclonal, chimeric or humanized.

As used herein, the term "chimeric" applied to an antibody or an antibody fragment means that two or more segments or portions of said antibody or antibody fragment derive from different animal species. A particular case of chimeric antibody is a "humanized" antibody, which comprises human framework regions with complementary determining regions (CDR) of another species. Technologies for the humanization of antibodies from non-human species are well-known for the one skilled in the art.

In a preferred embodiment of a medicament constituted of a penicillin-binding protein, or fragment, or variant, or variant fragment therof; or a nucleic acid; or an antibody or antibody fragment according to the invention, said penicillin-binding protein is *Neisseria meningitidis* PBP2.

In a particular embodiment, said penicillin-binding protein fragment is a fragment of *Neisseria meningitidis* PBP2 lacking the N-terminal transmembrane domain. In still a more particular embodiment, said fragment of *Neisseria meningitidis* PBP2 lacking the N-terminal transmembrane domain is purified from clone DH5pAA2 deposited on May 3, 2005 at the CNCM (Institut Pasteur, Paris, France) under number I-3422. The DH5pAA2 clone was obtained by cloning the penA gene of *Neisseria meningitidis* LNP8013 strain, deleted of the first 121 codons, between the Nde I and Xho I sites of the pET28b vector (Antignac A. et al. 2003. J Biol Chem. 278(34):31521-8).

In another preferred embodiment of a medicament constituted of a penicillin-binding protein, or fragment, or variant, or variant fragment therof; or a nucleic acid; or an antibody or antibody fragment according to the invention, said variant is a variant of a *Neisseria meningitidis* PBP expressed by *Neisseria gonorrhoeae* or *Streptococcus pneumoniae.* Such variants of a *Neisseria meningitidis* PBP expressed by *Neisseria gonorrhoeae* or *Streptococcus pneumoniae* include *Neisseria gonorrhoeae* PBP1 and PBP2, and *Streptococcus pneumoniae* PBP1b, PBP2a, and PBP2X. Preferably, said variant is *Neisseria* gonorrhoeae PBP2 or *Streptococcus pneumoniae* PBP2X.

The present invention further concerns a pharmaceutical composition comprising at least one penicillin-binding protein, or a fragment, or a variant, or a variant fragment thereof, or a nucleic acid, or an antibody or an antibody fragment according to the invention, with a pharmaceutically acceptable carrier or excipient. In the pharmaceutical composition, the penicillin-binding protein, or fragment, or variant, or variant fragment thereof, or the nucleic acid, or the antibody or antibody fragment may display any of the features previously described for a penicillin-binding protein, or nucleic acid, or antibody as a medicament according to the invention.

"Pharmaceutically acceptable" refers to a non-toxic, inert composition that is physiologically compatible with humans or other mammals.

Pharmaceutically acceptable carriers or excipients include dispersion agents, suspension agents, diluents, fillers, pH-adjusting and buffering agents, tonicity adjusting agents, wetting agents, preservatives, and the like.

The pharmaceutical compositions according to the invention may further comprise an adjuvant. The term "adjuvant" has its usual meaning in the art of vaccine technology, i.e. a substance or a composition of matter which is 1) not in itself capable of mounting a specific immune response against the immunogen of the vaccine, but which is 2) nevertheless capable of enhancing the immune response against the immunogen. A number of adjuvants and putative adjuvants (such as certain cytokines) share the ability to enhance the immune response. Suitable cytokines to be used as adjuvants are those which will normally also function as adjuvants in a vaccine composition, e.g. interferon γ (IFN-γ), Flt3 ligand (Flt3L), interleukin 1 (IL-1), interleukin 2 (IL-2), interleukin 4 (IL-4), interleukin 6 (IL-6), interleukin 12 (IL-12), interleukin 13 (IL-13), interleukin 15 (IL-15), and granulocyte-macrophage colony stimulating factor (GM-CSF). In the case of a medicament or a pharmaceutical composition comprising at least one nucleic acid encoding at least one PBP protein or fragment or variant or variant fragment thereof, said cytokine may also be encoded in the nucleic acid. Alternatively, the adjuvant may be a toxin, such as listeriolysin (LLO), lipid A and heat-labile enterotoxin. Also, a number of mycobacterial derivatives such as MDP (muramyl dipeptide), CFA (complete Freund's adjuvant) and the trehalose diesters TDM and TDE are interesting possibilities. Heat shock proteins may also be used as adjuvant, such as HSP70, HSP90, HSC70, GRP94, and calreticulin (CRT). In the case of a medicament or a pharmaceutical composition comprising at least one nucleic acid encoding at least one PBP protein or fragment or variant or variant fragment thereof, said heat shock protein may also be encoded in the nucleic acid.

Above-mentioned pharmaceutical compositions or medicaments may be administered by any route, including,but not limited to, oral, subcutaneous, intravenous, intranasal, transdermal, intraperitoneal, intramuscular, intrapulmonar, vaginal, rectal, or in any other acceptable manner.

Above-mentioned pharmaceutical compositions or medicaments may be formulated in a variety of forms, e.g. as a liquid, gel, lyophilized, or as a compressed solid. The preferred form will depend upon the particular indication being treated and will be apparent to one skilled in the art. In each case, the active ingredient or the combination of active ingredients to be administered should be formulated in a manner which will protect the active ingredient against degradation, in particular by enzymes.

Above-mentioned pharmaceutical compositions or medicaments may be co-administered, concurrently administered, and/or sequentially administered with one or more other therapeutic agents.

Above-mentioned pharmaceutical compositions or medicaments contain a therapeutically effective dose of active ingredient (i.e. at least one PBP or fragment or variant or variant fragment thereof, or at least one nucleic acid encoding at least one PBP or fragment or variant or variant fragment thereof, or at least one antibody or antibody fragment directed against at least one PBP). By "therapeutically effective dose" herein is meant a dose that is sufficient to produce the desired effects in relation to the condition for which it is administered. The exact dose will depend on the circumstances (the disease, the administration schedule, whether the pharmaceutical composition or medicament is administered alone or in conjunction with other therapeutic agents, the plasma half-life of the pharmaceutical composition or medicament, and the general health of the patient), and will be ascertainable by one skilled in the art using known techniques.

The present invention further concerns the use of at least one penicillin-binding protein or fragment, or variant, or variant fragment thereof according to the invention, for the preparation of a pharmaceutical composition for the vaccination of a mammal, preferably a human being, against infection by a bacterial strain expressing a *Neisseria meningitidis* PBP protein or a variant thereof. In such a use, the penicillin-binding protein, or fragment, or variant, or variant fragment thereof may display any of the features previously described for a penicillin-binding protein or fragment, or variant, or variant fragment thereof as a medicament according to the invention.

The invention also concerns the use of at least one nucleic acid according to the invention, for the preparation of a pharmaceutical composition for the vaccination of a mammal, preferably a human being, against infection by a bacterial strain expressing a *Neisseria meningitidis* PBP protein or a variant thereof. In such a use, said nucleic acid may display any of the features previously described for a nucleic acid as a medicament according to the invention.

By "vaccination" of a mammal against infection by a bacterial strain is meant an immunization of said mammal inducing protective immunity against said bacterial strain. By "immunization" is meant the stimulation of the immune system of said mammal, including the stimulation of the innate immune system, such as the complement system, dendritic cells, macrophages, neutrophils, eosinophils, and/or the adaptive immune system, such as T and B lymphocytes. The induction of "protective immunity" against infection by a bacterial strain means that the stimulation of the immune system induced by the immunization results in a state of immune resistance to said infectious bacterial strain. Said state of immune resistance to the infectious bacterial strain appears a few days after immunization and then persists for extended periods of time. The induction of protective immunity is thus useful in an already infected individual to help to jugulate the infection, or in a healthy individual to prevent a potential subsequent infection.

By "state of immune resistance", it is meant that the extent of the infection is significantly diminished when compared to a control mammal that did not receive said pharmaceutical composition. The extent of the bacterial infection may be determined by measuring bacteremia (cfu counts) by well-known technologies in presumably infected organs at several time points after infection. Preferably, the term "state of immune resistance" is used when the extent of bacterial infection is significantly reduced when compared to a control mammal that did not receive said pharmaceutical composition. Preferably, the extent of bacterial infection at 3-5 hours after infection is reduced of at least a 200-300 factor when compared to a control mammal that did not receive said pharmaceutical composition. Such a state of immune resistance may be induced for instance when the vaccination induces antibodies directed against at least one penicillin-binding protein expressed by *Neisseria meningitidis* or a variant thereof. Such antibodies include all classes of antibody, such as IgM, IgD, IgG, IgE ant IgA antibodies, as well as all subclasses such as IgG1, IgG2, IgG3, IgG4.

In all the description, the term "mammal" refers to any organism classified as a mammal, including mice, rats, rabbits, dogs, cats, cows, horses and humans. In one embodiment of the invention, the mammal is a mouse. In another embodiment of the invention, the mammal is a human.

The invention also concerns the use of at least one antibody or antibody fragment according to the invention, for the preparation of a pharmaceutical composition for the protection of a mammal, preferably a human being, against infection by a bacterial strain expressing a *Neisseria meningitidis* PBP protein or a variant thereof. In such a use, said antibody or antibody fragment may display any of the features previously described for an antibody or antibody fragment as a medicament according to the invention.

By "protection", it is meant that the extent of the bacterial infection is significantly diminished by the use of said pharmaceutical composition comprising one or several antibodies, or antibody fragments, when compared to a control mammal that did not receive said pharmaceutical composition. Preferably, the term "protection" is used when the extent of bacterial infection is significantly reduced when compared to a control mammal that did not receive said pharmaceutical composition. Preferably, the extent of bacterial infection at 3-5 hours after infection is reduced of at least a 200-300 factor when compared to a control mammal that did not receive said pharmaceutical composition.

In a preferred embodiment of a use according to the invention, said infectious bacterial strain is *Neisseria meningitidis.* In this case, said used pharmaceutical composition preferably vaccinates or protects said mammal against infection by all *Neisseria meningitidis* serogroups.

By "serogroup", it is meant a group of related microorganisms distinguished by its composition of antigens. More precisely, *Neisseria meningitidis* serogroups are defined according to their capsule antigen composition, which is determined using antibodies directed to capsule antigens. *Neisseria meningitidis* strains are divided into five main serogroups: A, B, C, Y and W135. Current vaccines against *Neisseria meningitidis* are based on capsular polysaccharide and are available only against strains belonging to serogroups A, C, Y and W135 but not against strains of serogroup B. In contrast, by a use according to the invention, it is possible to vaccinate or protect a mammal against all *Neisseria meningitidis* serogroups.

In another preferred embodiment of a use according to the invention, said infectious bacterial strain is a strain expressing a variant of a *Neisseria meningitidis* PBP protein, such as *Neisseria gonorrhoeae* and Streptococcus pneumonia.

In another preferred embodiment, when the infectious bacterial strain is *Neisseria meningitidis,* said pharmaceutical composition further vaccinates or protects said mammal against infection by a bacterial strain of a distinct species or genus expressing a variant of a *Neisseria meningitidis* PBP protein.

Preferably, such a bacterial strain of a distinct species or genus expressing a variant of a *Neisseria meningitidis* PBP protein is *Neisseria gonorrhoeae* or *Streptococcus pneumoniae.*

The invention also concerns the use of at least one penicillin-binding protein, or fragment, or variant or variant fragment thereof according to the invention, for the detection of antibodies directed against *Neisseria meningitidis* in a mammal, preferably a human being. In such a use, said penicillin-binding protein may display any of the features previously described for a penicillin-binding protein as a medicament according to the invention. Such a detection of antibodies directed against *Neisseria meningitidis* may be useful for instance to diagnose a *Neisseria meningitidis* infection in a non-vaccinated mammal, or to evaluate the efficiency of vaccination in a vaccinated mammal.

The detection may be performed using any of the many technologies known in the art for the detection of specific antibodies in serum using their purified specific antigen, such as for instance the ELISA technology.

The invention further relates to the use of at least one antibody or antibody fragment according to the invention, for the in vitro diagnosis from a biological sample of a mammal, preferably a human being, of *Neisseria meningitidis* infection. In such a use, said antibody or antibody fragment may display any of the features previously described for an antibody or antibody fragment as a medicament according to the invention.

The diagnosis may be performed using any of the many technologies known in the art for the detection of a specific antigen in serum using their antibodies or antibody fragments specific for this antigen, such as for instance the ELISA technology. The advantage of the use of at least one antibody or antibody fragment according to the invention is that it allows for the in vitro diagnosis of infection by a *Neisseria meningitidis* strain of any serogroup.

The invention also concerns a method of treatment of a mammal, preferably a human being, against *Neisseria meningitidis* infection and/or against infection by a bacterial strain expressing a *Neisseria meningitidis* PBP variant, comprising vaccinating said mammal against at least one penicillin-binding protein expressed by *Neisseria meningitidis,* or a variant thereof.

Vaccination may be performed by any technology known in the art, in particular by using a pharmaceutical composition comprising at least one penicillin-binding protein expressed by *Neisseria meningitidis,* or a fragment, or a variant, or a variant fragment thereof, or a nucleic acid encoding them, as described above.

The invention also concerns a method of treatment of a mammal, preferably a human being, against *Neisseria meningitidis* infection and/or against infection by a bacterial strain expressing a *Neisseria meningitidis* PBP variant, comprising injecting to said mammal an effective amount of a medicament or a pharmaceutical composition comprising at least one antibody or antibody fragment directed against at least one penicillin-binding protein expressed by *Neisseria meningitidis* or a fragment, or a variant, or a variant fragment thereof, as described above.

Having generally described this invention, a further understanding of characteristics and advantages of the invention can be obtained by reference to certain specific examples and figures which are provided herein for purposes of illustration only and are not intended to be limiting unless otherwise specified.

### DESCRIPTION OF THE DRAWINGS

**Figure 1.** Molecular Organization of PBPs of high molecular mass of class A (PBP1a of *E. coli,* PBP1 of *N. gonorrhoeae* and *Neisseiria meningitidis)* and of class B (PBP3 of'*E*. *coli,* PBP2 of *N. gonorrhoeae* and *Neisseiria meningitidis).* The conserved motifs (numbered from 1 to 9 in class A PBPs and from 1 to 7 in class B PBPs) as well as the distances between the motifs (number of amino acids) are represented (Goffin and Ghuysen, 1998. Microbiol. Mol. Biol. Rev. Dec; 62 (4) :1079-93.)

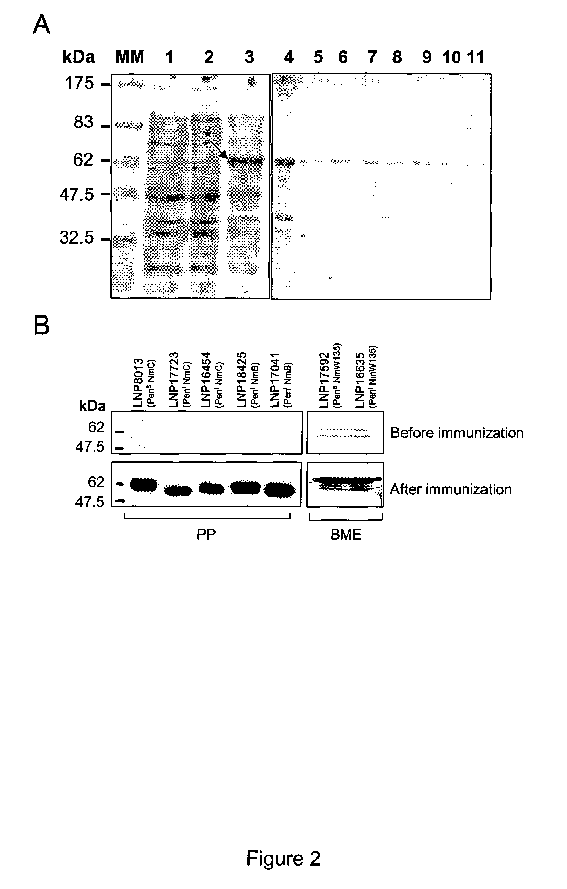

**Figure 2:** Specificity of serum of a rabbit immunized with PBP2 of *Neisseria meningitidis* strain LNP8013.
   **Figure 2A.** SDS-PAGE of solubilized whole cells from *E. coli* BL21(DE3) pLysS and eluted fractions containing purified His₆-tag-PBP2 protein of *Neisseria meningitidis* strain LNP8013. Solubilized cells were from *E. coli* BL21(DE3) pLysS harboring the expression vector alone (lane 1), the recombinant plasmid carrying the p*enA* gene without IPTG induction (lane 2) and after IPTG induction (lane 3). The arrow shows the band corresponding to the overexpressed recombinant PBP2 lacking the N-terminal transmembrane domain (121 aminoacids). Lane 4 corresponds to the soluble fraction that was passed trough the nickel column and lanes 5 to 11 represent the eluted fractions containing purified PBP2. **MM:** Molecular weight marker.
   **Figure 2B.** Western blot showing the binding of one rabbit serum obtained after immunization with the PBP2 protein of *Neisseria meningitidis* strain LNP8013. Rabbit serum was tested on purified His₆-tag-proteins (PP) corresponding to meningococcal strains LNP8013, LNP17723, LNP16454, LNP18425 and LNP17041, as well as bacterial membrane extracts (BME) from two serogroup W135 isolates (strains LNP17592 and LNP16635). NmC: serogroup C *Neisseiria meningitidis.* NmB: serogroup B *Neisseiria meningitidis.* NmW135: serogroup W135 *Neisseiria meningitidis.* Pen^{S}: susceptible strain to penicillin G. Pen^{I}: strain with intermediate susceptibility to penicillin G.

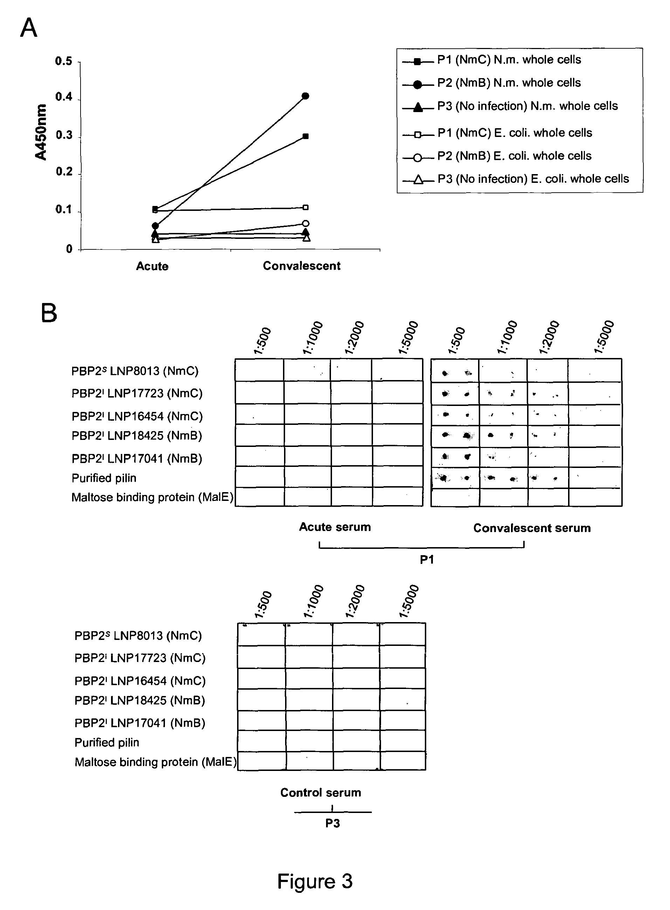

Figure 3: Immunogenicity of PBP2 during natural meningococcal infection.
   Figure 3A. IgG immune response against whole meningococcal cells (solid symbols) in pairs of sera from patients (P1 to P3) obtained at admission to hospital (acute sera) and 7-20 days after (convalescent sera). Patient P1 is a patient with confirmed meningococcal infection with a serogroup C *Neisseiria meningitidis.* Patient P2 is a patient with confirmed meningococcal infection with a serogroup B *Neisseiria meningitidis.* Patient P3 is a patient with no meningococcal infection. Open symbols represent the activity of each pair of sera against *E. coli* whole cells.
   **Figure 3B.** Dot blot analysis performed with the P1 pair of sera (acute and convalescent) and the control P3 serum (convalescent) against purified recombinant His₆-tag-PBP2s corresponding to Pen^{S} (PBP2^{S}) and Pen^{I} (PBP2^{I}) meningococcal isolates belonging to different serogroups and genetic lineages. Four different dilutions of sera were tested (1:500 to 1:5,000). For each protein, two concentrations (300 and 150 ng) were spotted, except for PBP2 from strain LNP8013 where 100 and 50 ng were used. Meningococcal pilin was purified as previously described (11).

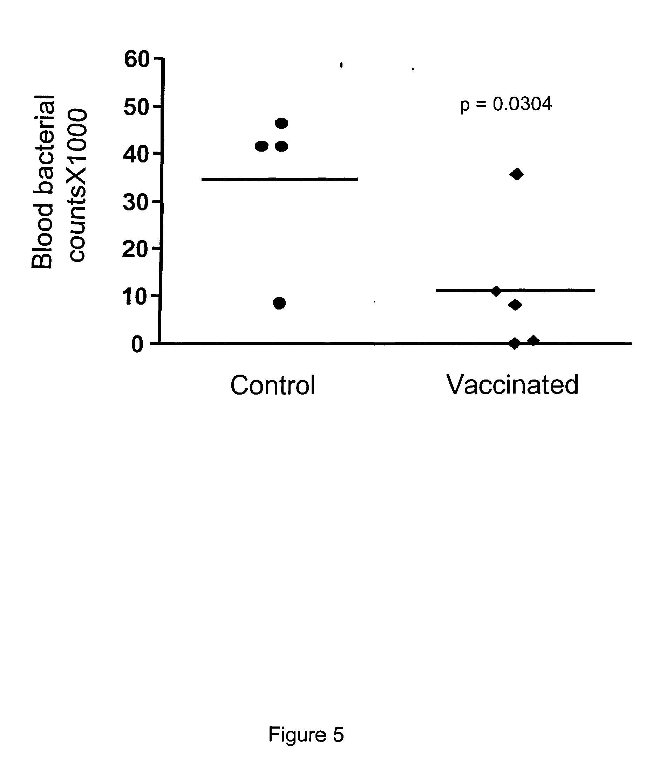

Figure 4: Passive protection studies against meningococcal bacteremia in mice challenged intraperitoneally. The protective role of the anti-PBP2 IgG was evaluated after i.p challenge with LNP8013 *Neisseiria meningitidis* strain harboring a wild type *penA*^{*S*} allele and three *penA*^{*I*} isogenic mutants, TR-TH41, TR16454, and TR16504. Purified rabbit IgG against PBP2 or CrgA were used. The used strain/IgG pairs are indicated above each curve. Solid and open symbols represent results from untreated control mice (PBS) and mice treated with 30 µg of IgG, respectively. Data are means ± S.D. of groups of three mice per time point from two independent experiments. Student's t test was used to compare numbers of CFU (log₁₀) between groups of untreated and treated mice. * *P* = or < 0.05.
**Figure 5**: Anti-meningococcal protection obtained in mice immunized with the PBP2 protein of penicillin susceptible *Neisseria meningitidis* serogroup C strain LNP8013. Vaccinated (n=5) and control mice (n=4) were challenged by i.p. infection with 5 x 10⁵ cfu of the penicillin susceptible meningococcal strain LNP8013. After three hours, cfu counts were determined in blood by plating serial blood dilutions on GCB plates. Results were normalised according to the initial inocula. Individual vaccinated mice (black rhombs) and control mice (black circles) are represented. A student's t-test was applied to determine the significance (p < 0.05) of the differences between both groups of mice.

### Example 1

### Production of recombinant Neisseria meningitidis PBP2 fragments lacking the N-terminal transmembrane domain

Modified *penA* genes lacking the sequence coding for the N-terminal transmembrane domain of PBP2 from several meningococcal Pen^{S} and Pen^{I} isolates were cloned into the pET28b expression vector (Novagen, Nottingham, UK).

*Escherichia coli* BL21 pLysS DE3 strain was then transformed with recombinant plasmids and His₆-tag-PBP2s were overexpressed under the control of the T7 bacteriophage promoter.

Purification of the recombinant proteins was performed using a nickel nitrilotriacetic acid-agarose column (Qiagen, Düren-Germany) as previously reported (Antignac, A., et al. 2003. J Biol Chem 278:31529-35).

After SDS-PAGE analysis the fractions containing purified PBP2 were pooled, dialyzed against phosphate-buffered saline, adjusted to 20% glycerol (v\v), and stored at-20°C.

Features of all *Neisseria meningitidis* strains for which a recombinant *Neisseria meningitidis* PBP2 fragment lacking the N-terminal transmembrane domain was produced are listed in following Table 3.

**Table 3. Neisseria meningitidis strains for which a recombinant Neisseria meningitidis PBP2 fragment lacking the N-terminal transmembrane domain was produced.**

| Strain | Serogroup | Serotype | Serosubtype^{a} | MIC (µg/ml) |
|---|---|---|---|---|
| | | | | Penicillin G^{b} |
| LNP8013 | C | NT | NST | 0.094 |
| LNP16454 | C | 2a | P1-5 | 1 |
| LNP16504 | B | NT | NST | 0.25 |
| LNP17041 | B | 1 | NST | 0.5 |
| LNP17723 | C | 2a | P1-5 | 0.75 |
| TR TH-41 | C | NT | NST | 0.5 |
| TR 16454 | C | NT | NST | 0.5 |
| TR 16504 | C | NT | NST | 0.38 |

| | | | | |
|---|---|---|---|---|
| ^{a} NT, nontypable; NST, nonsubtypable ^{b} MICs: susceptible < 0.125 µg/ml; resistant > 1 µg/ml | | | | |

Figure 2A shows the analysis of the different purification steps for a recombinant *Neisseria meningitidis* PBP2 fragment lacking the N-terminal transmembrane domain of penicillin susceptible *Neisseria meningitidis* serogroup C strain LNP8013.

### Example 2

### Binding capacities of a rabbit anti-PBP2 serum obtained after immunization with the recombinant Neisseria meningitidis PBP2 lacking the N-terminal transmembrane domain.

The anti-PBP2 serum was obtained after immunization of rabbits with the purified recombinant His₆-tag-PBP2 lacking the N-terminal transmembrane domain from the penicillin susceptible serogroup C strain LNP8013 (Figure 2A).

For PBP2 immunization, two 3-kg female rabbits were injected subcutaneously with 3 µg of the purified PBP2 supplemented with Freund complete adjuvant (Difco, MA) on day 0 and with Freund incomplete adjuvant on days 7 and 21. Pre-immune sera were obtained on day 0 before immunization and immune sera were collected on day 28.

To test the specificity of the rabbit anti-PBP2 sera, a western blot against *penA*^{*s*} and *penA*^{*I*} encoded PBP2s from meningococci belonging to different serogroups and genetic lineages was performed. Five thousand-fold dilution of sera permitted to show specific recognition by only immune sera.

The specificity of the rabbit anti-PBP2 sera was tested on the purified recombinant *Neisseria meningitidis* PBP2 fragments lacking the N-terminal transmembrane domain of penicillin described in Example 1, as well as on bacterial membrane extracts of two other *Neisseria meningitidis* strains belonging to serogroup W135 (strains LNP17592 and LNP16635).

The results show that the tested rabbit anti-PBP2 sera recognize PBP2s from several different meningococcal isolates belonging to serogroups B, C or W135, independently of the level of penicillin susceptibility of the different strains (Figure2B).

This confirms that the purified PBP2 protein is immunogenic in rabbit and that anti-PBP2 antibodies recognize PBP2s from several different meningococcal isolates, suggesting that they might have a protective effect if injected to a infected subject during *Neisseria meningitidis* infection by many different *Neisseria meningitidis* strains, including serogroup B strains.

### Example 3

### Immunogenicity of PBP2 during natural meningococcal infection.

Purified recombinant PBP2s corresponding to meningococcal isolates belonging to different genetic lineages, different serogroups (B and C), and various susceptibility to penicillin G, were spotted on nitrocellulose membrane and immuno-detected using a pair of sera from a patient (P1) with confirmed meningococcal infection (positive culture for a serogroup C *Neisseiria meningitidis).* The paired sera consisted of an acute serum obtained at admission to hospital and a convalescent serum obtained 10 days later. Serial serum dilutions were tested and IgG response was detected using a goat anti-human IgG (CALTAG laboratories, Burlingame CA). An IgG signal was only detected using convalescent serum against all tested *penA*^{*s*} and *penA*^{*I*} encoded PBP2s (Figure 3B).

Similar results were obtained when PBP2s were immunodetected with a pair of sera obtained from another patient (P2) with a confirmed serogroup B meningococcal infection. No signal was detected with both sera against an irrelevant protein (maltose binding protein, MalE of *E. coli*) while a positive signal was observed against a major meningococcal immunogenic protein (pilin) with the convalescent serum (Figure 3B).

This increase in anti-PBP2 immunoblotting paralleled the immune response of patients sera (dilution 1:1,000) tested by ELISA against *Neisseiria meningitidis* whole cells (Figure 3A).

No enhancement of signal was observed with a couple of control sera obtained from a patient (P3) with no meningococcal infection (Figure 3A). These data confirm the specificity of the immune response against PBP2 during natural meningococcal infection, suggesting that *Neisseria meningitidis* PBP2 should be a convenient target for protective antibodies during *Neisseria meningitidis* infection.

### Example 4

### Passive protection studies against meningococcal bacteremia in mice challenged intraperitoneally.

Next, the ability of a rabbit anti-PBP2 IgG to confer passive protection against *Neisseiria meningitidis* bacteremia in a murine intraperitoneal (i.p.) model of infection was tested.

Anti-PBP2 IgG was obtained by purification of total IgG from one rabbit immune serum after immunization by the purified recombinant His₆-tag-PBP2 lacking the N-terminal transmembrane domain from the penicillin susceptible serogroup C strain LNP8013. Total IgG were first precipitated with 33% (NH₄)₂SO₄ and dialyzed against phosphate saline buffer 17.5 mM, pH 6.3. This fraction was adsorbed on a cianogen bromide activated Sepharose 4B column (Pharmacia, Sweden) previously coated with a bacterial extract of BL21 pLysS DE3 *E. coli* harboring the pET28B expression vector. Protein concentration in the eluted fraction was determined by absorbance at 280nm.

For passive protection experiments, all tested meningococcal strains were passaged in mice to maintain virulence and stored at -70 °C in GC-broth with 30% of glycerol. They were grown overnight at 37 °C in 5% CO₂ on GCB agar plates and were then inoculated into BHI broth. Bacteria were grown for 4 h to reach the exponential phase and adjusted at 10⁶cfu/ml.

Five-week-old female BALB/c mice (Harlan, France) were treated at time 0 with an intravenous administration of 30 µg, 3 µg and 0.3 µg of purified anti-PBP2 rabbit IgG. These doses were calculated to roughly achieve in mice a concentration (0.3 µg/ml to 30 µg/ml) of specific IgGs similar to that expected in humans (about 0.3 to 0.5 % of total IgGs). For each experiment, control mice were injected with PBS or with a purified rabbit IgG against the meningococcal cytoplasmic (thus not accessible to antibodies) regulatory protein, CrgA (Deghmane, A. E., and M. K. Taha. 2003. Mol Microbiol 47:135-43).

Two hours later mice were challenged i.p. with 0.5 ml (5 x 10⁵ cfu) of the serogroup *C Neisseiria meningitidis* strain LNP8013, a penicillin susceptible strain harboring a wild type *penA*^{*s*} allele. Blood cfu counts from three mice and from two independent experiments were performed 1h, 2h, 3h and 4h after the bacterial challenge. There was no significant difference in the level of bacteremia after 1 and 2 hours post challenge among mice (Figure 4). However anti-PBP2 rabbit IgG-injected mice showed a reduction in bacteremia after 3 and 4 hours when compared to controls and this reduction was dose-dependent for the three used doses (30 µg, 3 µg and 0.3 µg) (Figure 4). No protection was observed for the negative controls, i.e. mice injected with PBS or with purified rabbit IgG against the meningococcal cytoplasmic regulatory protein, CrgA (Figure 3) (Deghmane, A. E., and M. K. Taha. 2003. Mol Microbiol 47:135-43).

Next, the ability of the anti-PBP2 IgG to passively protect against i.p. challenge with three LNP8013 isogenic mutants with reduced susceptibility to penicillin was analyzed. All these strains have alterations within the 3' transpeptidase-encoding region of the *penA* gene. The TR-TH41, TR16454 and TR16504 strains were obtained by transformation of LNP8013 with the PCR amplified *penA* allele from three different meningococcal strains (TH-41, LNP16454 and LNP16504, respectively) (Antignac, A., et al. 2003. J Biol Chem 278:31529-35). As observed in Figure 4, the anti-PBP2 IgG reduced bacteremia after challenge with all three *penA* mutants, despite alterations in the PBP2 protein they express.

These results demonstrate that an injection of antibodies binding a particular *Neisseria meningitidis* strain PBP2 protein is sufficient to induce a protection of a mouse infected by this particular strain, but also by mutant *Neisseria meningitidis* strains. It thus suggests that the injection of antibodies binding a particular *Neisseria meningitidis* strain PBP2 protein should be sufficient to induce a protection against infection by many distinct *Neisseria meningitidis* strains, including *Neisseria meningitidis* strains of other serogroups.

### Example 5

### Vaccination of mice with a fragment of Neisseria meningitidis PBP2 protein

### Methods

Five female BALB/c mice that were 6 weeks old were immunised with 12 µg of the purified recombinant His₆-tag-PBP2 lacking the N-terminal transmembrane domain from the penicillin susceptible serogroup C strain LNP8013 (PBP2C^{S}). Four control mice were injected with saline solution. Injections were performed subcutaneously on days 0, 7 and 14 using complete Freund's adjuvant (Difco, Detroit, MI, USA) for the first dose and incomplete Freund's adjuvant for the second one. On day 28 mice were bled to test immune response by ELISA using 96-well PBP2C^{S} coated plates with 1 µg of the purified PBP2. Intraperitoneal (i.p.) challenge with 5 x 10⁵ cfu of strain LNP8013 (Pen^{S}) was then performed. Blood colony forming units (cfu) counts were performed on GCB plates 3h after the bacterial challenge.

### Results

A good level of anti-PBP2 antibodies titres was observed by ELISA as compared to control mice. No difference in antibody titre was observed among vaccinated mice.

Vaccinated mice showed significant lower cfu counts in blood when compared to control mice (P=0.0304) (Figure 5). These results suggest that purified recombinant His₆-tag-PBP2 lacking the N-terminal transmembrane domain from the penicillin susceptible serogroup C strain LNP8013 (PBP2C^{S} is immunogenic and is able to induce a protective response against *Neisseiria meningitidis.*

Overall, the results described in the previous examples clearly show that *Neisseria meningitidis* PBP2 protein is immunogenic during normal human *Neisseria meningitidis* infection and that immunization of mice with a PBP2 fragment induces anti-PBP2 antibodies.

In addition, such an immunization induces protective immunity in vaccinated mice.

Most of all, despite the high genetic variability of *Neisseria meningitidis* PBP2 protein, the results show that antibodies directed against a particular *Neisseria meningitidis* PBP2 protein are able to bind *Neisseria meningitidis* PBP2 proteins of other different *Neisseria meningitidis* strains, and that such antibodies confer a protection if injected during a *Neisseria meningitidis* infection.

The ability of antibodies directed against a particular *Neisseria meningitidis* PBP2 protein to bind *Neisseria meningitidis* PBP2 proteins of other different *Neisseria meningitidis* strains is probably linked to the presence of the conserved amino acid motifs (Sx₂K, SxN, and KTG) in the PB module. These motifs being highly conserved among PBP proteins, either among mutants of a particular PBP protein or even among different bacteria groups, this strategy should thus be also applicable to vaccinates mammals against infection by bacterial strains of distinct species expressing a variant of a *Neisseria meningitidis* PBP protein.

PBP proteins expressed by *Neisseria meningitidis* could thus be used for vaccination against *Neisseria meningitidis* and/or bacterial strains of dictinct species expressing variants of *Neisseria meningitidis* PBP proteins. Antibodies directed against *Neisseria meningitidis* PBP proteins could be used for the therapeutic treatment of individuals infected by *Neisseria meningitidis* and/or bacterial strains of distinct species expressing variants of *Neisseria meningitidis* PBP proteins. To increase the protection effect of PBP proteins or antibodies administrations, a combination of several PBP proteins or antibodies may be used.

## Claims

1. A penicillin-binding protein expressed by *Neisseria meningitidis,* or a fragment, or a variant, or a variant fragment thereof, as a medicament.

2. A nucleic acid encoding at least one penicillin-binding protein expressed by *Neisseria meningitidis,* or a fragment, or a variant, or a variant fragment thereof, as a medicament.

3. An antibody or an antibody fragment directed against a penicillin-binding protein expressed by *Neisseria meningitidis,* or a variant, or a fragment, or a variant fragment thereof, as a medicament.

4. A medicament according to any of claims 1 to 3, wherein said penicillin-binding protein is *Neisseria meningitidis* PBP2.

5. A medicament according to claim 4, wherein said penicillin-binding protein fragment is a fragment of *Neisseria meningitidis* PBP2 lacking the N-terminal transmembrane domain.

6. A medicament according to claim 5, wherein said fragment of *Neisseria meningitidis* PBP2 lacking the N-terminal transmembrane domain is purified from clone DH5pAA2 deposited on May 3, 2005 at the CNCM under number 1-3422.

7. A medicament according to any of claims 1 to 3, wherein said variant is a variant of a *Neisseria meningitidis* PBP expressed by *Neisseria gonorrhoeae* or *Streptococcus pneumoniae.*

8. A medicament according to claim 7, wherein said variant is *Neisseria gonorrhoeae* PBP2 or *Streptococcus pneumoniae* PBP2X.

9. A pharmaceutical composition comprising at least one penicillin-binding protein, or fragment, or variant, or variant fragment thereof; or at least one nucleic acid; or at least one antibody or antibody fragment according to any of claims 1 to 8, with a pharmaceutically acceptable carrier or excipient.

10. Use of at least one penicillin-binding protein or fragment, or variant, or variant fragment thereof, according to any of claims 1 and 4 to 8, for the preparation of a pharmaceutical composition for the vaccination of a mammal, preferably a human being, against infection by a bacterial strain expressing a *Neisseria meningitidis* PBP protein or a variant thereof.

11. Use of at least one nucleic acid according to any of claims 2 and 4 to 8, for the preparation of a pharmaceutical composition for the vaccination of a mammal, preferably a human being, against infection by a bacterial strain expressing a *Neisseria meningitidis* PBP protein or a variant thereof.

12. Use of at least one antibody or antibody fragment according to any of claims 3 to 8, for the preparation of a pharmaceutical composition for the protection of a mammal, preferably a human being, against infection by a bacterial strain expressing a *Neisseria meningitidis* PBP protein or a variant thereof.

13. The use of any of claims 10 to 12, wherein said infectious bacterial strain is *Neisseria meningitidis.*

14. The use of claim 11, wherein said pharmaceutical composition vaccinates or protects said mammal against infection by all *Neisseria meningitidis* serogroups.

15. The use of any of claims 10 to 12, wherein said infectious bacterial strain is *Neisseria gonorrhoeae* or *Streptococcus pneumoniae.*

16. The use of any of claims 13 and 14, wherein said pharmaceutical composition further vaccinates or protects said mammal against infection by a bacterial strain of a distinct species or genus expressing a variant of a *Neisseria meningitidis* PBP protein.

17. The use of claim 16, wherein said bacterial strain of a distinct species or genus expressing a variant of a Neisseria meningitidis PBP protein is Neisseria *gonorrhoeae* or Streptococcus *pneumoniae.*

18. Use of at least one penicillin-binding protein, or fragment, or variant, or variant fragment thereof according to any of claims 1 and 4 to 8, for the detection of antibodies directed against *Neisseria meningitidis* in a mammal, preferably a human being.

19. Use of at least one antibody or antibody fragment according to any of claims 3 to 8, for the in vitro diagnosis from a biological sample of a mammal, preferably a human being, of *Neisseria meningitidis* infection.
